(19) Europäisches Patentamt
European Patent Office
Office européen des brevets

(11) EP 1 848 507 B1

(12) EUROPÄISCHE PATENTSCHRIFT

(45) Veröffentlichungstag und Bekanntmachung des Hinweises auf die Patenterteilung:
**05.08.2009 Patentblatt 2009/32**

(21) Anmeldenummer: **06700808.6**

(22) Anmeldetag: **20.01.2006**

(51) Int Cl.:
***A61Q 17/04*** (2006.01) ***A61K 8/11*** (2006.01)
***A61K 8/04*** (2006.01) ***A61K 8/37*** (2006.01)
***A61K 8/25*** (2006.01) ***A61K 8/29*** (2006.01)

(86) Internationale Anmeldenummer:
**PCT/EP2006/000517**

(87) Internationale Veröffentlichungsnummer:
**WO 2006/087066 (24.08.2006 Gazette 2006/34)**

(54) **ZUBEREITUNG ENTHALTEND NANOPARTIKULÄRES UV-SCHUTZMITTEL**

PREPARATION CONTAINING NANOPARTICULATE UV PROTECTION AGENTS

COMPOSITION CONTENANT UN AGENT ANTI-UV NANOPARTICULAIRE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **17.02.2005 DE 102005007482**

(43) Veröffentlichungstag der Anmeldung:
**31.10.2007 Patentblatt 2007/44**

(73) Patentinhaber:
- **Merck Patent GmbH**
  **64293 Darmstadt (DE)**
- **Sachtleben Chemie GmbH**
  **47198 Duisburg (DE)**

(72) Erfinder:
- **PFLUECKER, Frank**
  **64297 Darmstadt (DE)**
- **BECK, Joem**
  **64342 Seeheim-Jugenheim (DE)**
- **HIRTHE, Bernd**
  **47918 Toenisvorst (DE)**
- **JOHN, Stephan**
  **47198 Duisburg (DE)**
- **SCHULZE, Nicole**
  **47057 Duisburg (DE)**

(56) Entgegenhaltungen:
**EP-A- 0 988 853** **EP-A- 1 287 807**
**WO-A-02/22098** **WO-A-20/05019348**
**US-A- 4 927 464** **US-A1- 2003 099 721**
**US-A1- 2003 104 198**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 848 507 B1

Printed by Jouve, 75001 PARIS (FR)

## Beschreibung

Die vorliegende Erfindung betrifft ölige Dispersionen nanopartikulärer UV-Schutzmittel, deren Herstellung und Verwendung. Die vorliegende Erfindung betrifft weiter neue Zusammensetzungen zur topischen Anwendung, die insbesondere zum Lichtschutz der Haut und/oder der Haare gegen UV-Strahlung bestimmt sind (Zusammensetzungen, die im folgenden einfach als Sonnenschutzmittel bezeichnet werden), sowie ihre Verwendung bei der obengenannten kosmetischen Anwendung.

Es ist bekannt, dass durch Lichtstrahlung mit einer Wellenlänge im Bereich von 280 bis 400 nm die menschliche Epidermis gebräunt werden kann und dass Strahlung mit einer Wellenlänge im Bereich von 280 bis 320 nm, die unter Bezeichnung UV-B bekannt ist, Erytheme und Hautverbrennungen hervorruft, die der Ausbildung von natürlicher Bräune abträglich sein können. Die UV-B-Strahlung sollte deshalb ausgefiltert werden.

Es ist ferner bekannt, dass UV-A-Strahlung mit einer Wellenlänge im Bereich von 320 bis 400 nm, die die Haut bräunt, eine Veränderung der Haut hervorrufen kann, insbesondere im Falle von empfindlicher Haut oder Haut, die kontinuierlich Sonnenstrahlung ausgesetzt ist. UV-A-Strahlung bewirkt insbesondere einen Verlust der Elastizität der Haut und das Auftreten von Falten, was zu einer vorzeitigen Alterung führt. Sie begünstigt das Auslösen einer Erythembildung oder verstärkt diese Reaktion bei manchen Personen, und sie kann sogar die Ursache für durch Licht ausgelöste toxische oder allergische Reaktionen sein. Es ist daher wünschenswert, auch die UV-A-Strahlung auszufiltern.

In der Kosmetik wurden bis jetzt zahlreiche organische Sonnenschutzfilter angegeben, welche die schädliche UV-A-Strahlung mehr oder weniger selektiv absorbieren können.

Eine diesbezüglich besonders interessante Gruppe von UV-A-Filtem besteht gegenwärtig aus den Dibenzoylmethanderivaten, insbesondere 4,4'-Methoxy-tert.-butyldibenzoylmethan, die ein starkes intrinsisches Absorptionsvermögen aufweisen. Diese Dibenzoylmethanderivate, die derzeit als im UV-A-Bereich wirksame Filter an sich wohlbekannte Produkte sind, sind insbesondere in den französischen Patentanmeldungen FR-A-2 326 405 und FR-A-2 440 933 sowie in der europäischen Patentanmeldung EP-A-0114 607 beschrieben. Das 4,4'-Methoxy-tert.-butyldibenzoylmethan ist im Übrigen gegenwärtig unter der Handelsbezeichnung Eusolex® 9020 von der Fa. Merck im Handel erhältlich.

Diese Dibenzoylmethanderivate können mit einem UV-B-Filter kombiniert werden, um einen vollständigen Schutz über das gesamte Spektrum des Sonnenlichts im UV-Bereich zu erhalten.

Es ist ferner bekannt, dass durch die Zugabe eines anorganischen Pigments und insbesondere eines Pigments von Titandioxid ($TiO_2$) die Lichtschutzeigenschaften der Sonnenschutzmittel, die UV-Filter enthalten, verbessert werden können.

Deshalb ist die Kombination von Dibenzoylmethanderivaten und nanopartikuläre Metalloxiden von Metalloxiden auf dem Gebiet der Sonnenschutzmittel sehr geschätzt.

Es zeigt sich aber, dass die Kombinationen von Dibenzoylmethanderivaten und anorganischen nanopartikuläre Metalloxiden und insbesondere die Kombination von 4,4'-Methoxy-tert.-butyldibenzoylmethan und Metalloxiden mehrere Nachteile aufweisen, die sich nicht nur auf die Art und damit die Qualität der Produkte, die sie enthalten, sondern auch auf ihre Attraktivität für die Verbraucher auswirkt. Bei den Zusammensetzungen, die diesen Typ von Kombination enthalten, wird zum einen häufig ein verstärkter Abbau von Dibenzoylmethanderivaten in Formulierungen beobachtet, wenn Titandioxid-Partikel anwesend sind Zum anderen entsehen in kosmetischen Formulierungen, welche diese Kombination enthalten immer wieder Schwierigkeiten durch Auskristallisation von komplexen des Dibenzoylmethanderivats. Weiter wird häufig eine Farbänderung beobachtet, die in einer mehr oder weniger intensiven Gelb- oder Rotfärbung der Formulierungen zum Ausdruck kommt. Abgesehen davon, dass dieses Phänomen das Lichtschutzvermögen der Dibenzoylmethanderivate und insbesondere des 4,4'-Methoxy-tert.-butyldibenzoylmethan vermindert, ist diese Färbung aus kosmetischer Sicht natürlich nicht wünschenswert.

Es wird ferner beobachtet, dass diese Phänomene im Falle der Nanopigmente von $TiO_2$ besonders ausgeprägt sind.

Im Stand der Technik wurden bereits verschiedene Versuche, einzelne dieser Probleme zu lösen, angegeben: In der japanischen Patentanmeldung JP61-215314 wurde die Verwendung von Maskierungsmitteln empfohlen, welche unter Edetinsäure, Metaphosphorsäure, Polyphosphorsäure und/oder den Salzen dieser Säuren ausgewählt sind, um das Phänomen der Gelbfärbung zu vermindern. Diese Lösung ist jedoch nicht völlig zufriedenstellend.

In der Europäischen Patentanmeldung EP-A-0 748 624 wird festgestellt, dass die Verwendung von nanopartikuläre Metalloxiden von Titandioxid, die mit einem Silicon (Silanderivat oder Siloxanderivat) behandelt sind, die Gelbfärbung, die üblicherweise bei den Sonnenschutzmitteln beobachtet wird, welche herkömmliche Kombinationen vom Typ Dibenzoylmethanderivat/Pigment von $TiO_2$ enthalten, deutlich verringert.

Weiter sind aus der Druckschrift WO-A-94/04131 gegenüber Licht stabile Filterzusammensetzungen bekannt, die in wohldefinierten Mengenanteilen ein Dibenzoylmethanderivat in Kombination mit einem Benzylidencampherderivat enthalten. Nach dieser Druckschrift kann das Dibenzoylmethanderivat durch den Benzylidencampher in den angegebenen Mengenanteilen gegenüber Licht stabilisiert, d.h. seine Zersetzung unter der Einwirkung von UV-Strahlung und insbesondere UV-A-Strahlung eingeschränkt werden. In der gleichen Druckschrift ist angegeben, dass diese photostabilen Zusammensetzungen ferner ein organisches Pigment, das UV-Strahlung abblockt, und insbesondere ein Pigment von Titandioxid enthalten können, das mit einer Verbindung und insbesondere mit einer siliconhaltigen Verbindung umhüllt sein kann.

Trotz dieser Versuche, die oben genannten Probleme bei Kombination von Dibenzoylmethanderivaten mit Metalloxid-Partikeln zu lösen, besteht nach wie vor Bedarf nach einer Metalloxid-Qualität, die alle genannten Probleme gleichzeitig In befriedigender Weise löst.

In der Deutschen Patentanmeldung DE 10333029 wird ein nanopartikuläres UV-Schutzmittel, welches eine Siliciumdioxid-Beschichtung aufweist und erhältlich ist durch hydrothermale Behandlung eines nanopartikulären Metalloxids und anschließendes Aufbringen einer Siliciumdioxid-Beschichtung beschrieben, das sich mit Dibenzoylmethan-Derivaten kombinieren läßt. Bevorzugt ist dabei ein Titandioxid mit einem Siliciumdioxidgehalt bezogen auf das gesamte nanopartikuläre UV-Schutzmittel von 12 bis 20 Gew.-%.

Es hat sich jedoch gezeigt, dass es für die Anwendung in kosmetischen Zubereitungen wünschenswert ist, das nanopartikuläre Metalloxid direkt in einer vordispergierten Form vorliegen zu haben.

Gegenstand der Erfindung ist eine Ölige Dispersion eines nanopartikulären UV-Schutzmittels, welches eine Sillciumdioxid-Beschlchtung aufweist, **dadurch gekennzeichnet, dass** die Dispersion 30 bis 70 Gew.-% eines Esters von gesättigten unverzweigten Alkancarbonsäuren einer Kettenlänge von 6 bis 12 C-Atomen mit Butylenglykol und
2 bis 15 Gew.-% Dispergierhilfsmittel enthaltend Polyglyceryldipolyhydroxystearat und
25 bis 60 Gew.-% nanopartikuläres UV-Schutzmittel mit Siliciumdioxid-Beschichtung enthält.

Als Dispergierhilfsmittel wird dabei erfindungsgemäß ein Polyglyceryldipolyhydroxystearat eingesetzt, wobei das Dispergierhilfsmittel vorzugweise in Mengen von 2,5 bis 10 Gew.-%, insbesondere bevorzugt in Mengen von 3 bis 6 Gew.-% bezogen auf die gesamte Dispersion in der Dispersion enthalten ist Ganz besonders bevorzugt ist erfindungsgemäß dabei der Einsatz von Polyglyceryl-12-dipolyhydroxystearat, das unter der Bezeichnung Dehymuls® PGPH (Fa. Cognis) erhältlich ist.

Erfindungsgemäß wird ein Ester von gesättigten unverzweigten Alkancarbonsäuren einer Kettenlänge von 6 bis 12 C-Atomen mit Butylenglykol und insbesondere bevorzugt Butylenglykoldicaprylat/dicaprat eingesetzt. Butylenglykoldicaprylat/dicaprat ist beispielsweise unter der Bezeichnung Migylol® 8810 (Fa. Sasol) erhältlich. Der Ester ist dabei vorzugsweise in Mengen von 40 bis 65 Gew.-%, insbesondere bevorzugt in Mengen von 50 bis 60 Gew.-% bezogen auf die gesamte Dispersion, in der Dispersion enthalten.

Ein erfindungsgemäß bevorzugt einzusetzendes nanopartikuläres UV-Schutzmittel ist dabei erhältlich durch hydrothermale Behandlung eines nanopartikulären Metalloxids und anschließendes Aufbringen einer Siliciumdioxid-Beschichtung eingesetzt wird, wobei es sich bei dem Metalloxid vorzugsweise im wesentlichen um Titandioxid handelt, das gegebenenfalls mit Eisen dotiert sein kann, wobei das Titandioxid mit Siliciumdioxid-Beschichtung in der Dispersion vorzugsweise in Mengen von 30 bis 50 Gew.-%, insbesondere bevorzugt in Mengen von 33 bis 40 Gew.-% bezogen auf die gesamte Dispersion, in der Dispersion enthalten ist.

Entsprechende nanopartikuläre UV-Schutzmittel sind dabei In DE 10333029 beschrieben. Als Hydrothermalbehandlung wird dabei das Erhitzen einer wässrigen Lösung, bzw. Suspension oder Dispersion in einem geschlossenen Behälter, gegebenenfalls unter Druck, bezeichnet (vgl. auch Ullmanns Enzyklopädie der Technischen Chemie, 4. Auflage, 1978, Band 15, S.117 ff: K.Recker, Einkristallzüchtung).

Unter einem nanopartikulären UV-Schutzmittel wird im Sinne der vorliegenden Erfindung vorzugsweise ein nanopartikuläres Metalloxid mit Siliciumdioxid-Beschichtung verstanden. Die Kristallitgröße des nanopartikulären Metalloxides in dem nanopartikulären UV-Schutzmittel bestimmt nach der Scherrer-Methode liegt üblicherweise im Bereich von 5 nm bis 100 nm, vorzugsweise im Bereich 8 bis 50 nm und insbesondere bevorzugt unterhalb von 25 nm. Die im Transmissionselektronenmikroskop ermittelbaren Abmessungen des nanopartikulären Metalloxides liegen üblicherweise bei einer Länge von 5 bis 150 nm und einer Breite von 5 bis 60 nm. Vorzugsweise liegt die Länge im Bereich von 20 bis 60 nm und die Breite im Bereich von 8 bis 30 nm.

Als nanopartikuläre Metalloxide kommen dabei für den erfindungsgemäßen Einsatz insbesondere Titandioxid, Eisenoxide, Zinkoxid oder auch Ceroxide zum Einsatz, wobei Titandioxid als Metalloxid erfindungsgemäß insbesondere bevorzugt ist, da es die erfindungsgemäßen Aufgaben in besonderer Weise erfüllt. Titandioxid kann dabei in Form von Rutil oder Anatas oder in amorpher Form, vorzugsweise aber in Form von Rutil und/oder Anatas, vorliegen. Bevorzugte Primärpartikelgröße liegt im Bereich vom 5 bis 50 nm. Dabei sind die Primärpartikel insbesondere bei Anatas vorzugsweise rund, während Rutil-Primärpartikel häufig in Nadel- oder Spindelform bis hin zu Ovalen ("eiförmig") auftreten. Es können erfindungsgemäß jedoch auch runde Rutil-Primärpartikel eingesetzt werden.

Die Siliciumdioxid-Beschichtung soll das nanopartikulären Metalloxid möglichst vollständig bedecken, und da es als UV-Filter jedoch inert ist, dennoch nicht in zu großen Mengen vorliegen. Es hat sich gezeigt, dass es vorteilhaft ist, wenn der Siliciumdioxidgehalt bezogen auf das gesamte nanopartikuläre UV-Schutzmittel 5 bis 50 Gew.-%, vorzugsweise 8 bis 30 Gew.-% und insbesondere bevorzugt 12 bis 20 Gew.-% beträgt.

Das resultierende nanopartikuläre UV-Schutzmittel zeigt üblicherweise eine Partikelgröße nach der Scherrer-Methode im Bereich von 5 nm bis 100 nm, vorzuzugsweise im Bereich 8 bis 50 nm und insbesondere bevorzugt unterhalb

von 25 nm. Die im Transmissionselektronenmikroskop ermittelbaren Abmessungen des nanopartikulären UV-Schutzmittels liegen üblicherweise bei einer Länge von 5 bis 160 nm und einer Breite von 10 bis 70 nm. Vorzugsweise liegt die Länge im Bereich von 30 bis 70 nm und die Breite im Bereich von 18 bis 40 nm.

Insbesondere bevorzugt ist dabei erfindungsgemäß der Einsatz von dem Siliciumdioxid-beschichteten Titandioxid, das unter der Bezeichnung Eusolex®T-AVO (Fa. Merck KGaA) erhältlich ist.

Die erfindungsgemäße ölige Dispersion nanopartikulärer UV-Schutzmittel zeigt dabei vorteilhafte Eigenschaften gegenüber dem Stand der Technik hinsichtlich:

- UV-Absorption, insbesondere Breitband- bzw. UV-A- und auch UV-B-Absorption,
- Transparenz im sichtbaren Licht (VIS),
- gute, insbesondere erhöhte Photostabilität,
- verringerte bzw. verhinderte Photoaktivität,
- hydrophile Oberfläche, gute Einarbeitung und Absetzstabilität in wässrigen Phasen;
- leichte Einarbeitung in Emulsionen, insbesondere Öl-in-Wasser-Emulsionen,
- niedrige Dispergierhilfsmittelkonzentration bei hohem UV-Schutzmittel-Gehalt,
- gute Lagerstabilität in bevorzugten Ausführungsformen verbunden mit thixotropem Verhalten,
- ein hohes Aufnahmevermögen für öllösliche, bei 25°C feste organische UV-Filter in dem Öl,
- sehr geringes Allergiepotential und damit verbunden insbesondere auch Eignung für Lichtschutzmittel für Kinder bzw. Kleinkinder,
- in Kombinaton mit Dibenzoylmethan-Derivaten, insbesondere:
    - ○ verringerte Verfärbung der Formulierung und/oder
    - ○ nachlassende Verfärbung der Formulierung während der Lagerung und/oder
    - ○ keine oder reduzierte Auskristallisation von Komplexen der Dibenzoylmethan-Derivate und/oder
    - ○ erhöhte Lagerstabilität der Dibenzoylmethan-Derivate und/oder
    - ○ verbesserte Lichtschutzwirkung, insbesondere nach Lagerung,
- in Kombination mit Selbstbräunem, insbesondere Dihydoxyaceton, wird keine oder eine gegenüber dem stand der Technik verminderte Destabilisierung des Selbstbräuners beobachtet,
- in Kombination mit Benzophenon-Derivaten, insbesondere 2-Hydroxy-4-Methoxy-Benzophenon, wird eine Stabilisierung der Benzophenon-Derivate beobachtet.

Die hervoragende UV-Absorption der erfindungsgemäßen öligen Dispersion zeigt sich dabei insbesondere im sogenannten Boots Star Rating, dessen Bestimmung in T. Rudolf "UV-A-Protection assessment of Sunscreens - a critical review on the UV-A- to UV-B-absorbance radio", SÖFW-Journal 130 (9), 2004, S.28ff. beschrieben ist.

Es hat sich dabei gezeigt, dass die erfindungsgemäßen Vorteile in besonderer Weise bei ölige Dispersionen eines nanopartikulären UV-Schutzmittels mit Siliciumdioxid-Beschichtung enthaltend 30 bis 70 Gew.-% mindestens eines Esters von gesättigten unverzweigten Alkancarbonsäuren einer Kettenlänge von 6 bis 12 C-Atomen mit Butylenglykol, vorzugsweise Butylenglykoldicaprylat/dicaprat, 2 bis 15 Gew.-Polyglyceryldipolyhydroxystearat und 25 bis 60 Gew.-% eines nanopartikulären Titandioxids mit Siliciumdioxid-Beschichtung, jeweils bezogen auf die gesamte Dispersion, verwirklicht sind.

Dabei hat es sich insbesondere gezeigt, dass es zur gleichzeitigen Verwirklichung der oben genannten Vorteile vorteilhaft sein kann, wenn das nanopartikuläre Metalloxid mit Cer oder Eisen, vorzugsweise Eisen, dotiert ist.

In einer anderen ebenfals bevorzugten Variante der vorliegenden Erfindung ist das nanopartikuläre Metalloxid jedoch frei von Dotierstoffen.

Die nachlassende Verfärbung der Formulierung während der Lagerung bei Kombination mit Dibenzoylmethanderivaten zeigt sich dabei bei allen üblichen Lagertemperaturen für kosmetische Formulierungen, insbesondere bei 4°C, Raumtemperatur und 50°C. Diese positive Wirkung beginnt direkt nach Herstellung der Formulierung. Eine erneute Intensivierung der Verfärbung tritt - soweit bislang bekannt - in der üblichen Lebensdauer einer kosmetischen Formulierung nicht ein.

Es hat sich weiter gezeigt, dass es von Vorteil sein kann, wenn die erfindungsgemäße Dispersion mindestens einen bei 25°C festen, öllöslichen, organischen UV Filter enthält. Bei der Herstellung von Lichtschutzzubereitungen ist die Einarbeitung dieser organischen UV-Filter oft eine formulierungstechnische Hürde. Die erfindungsgemäße ölige Dispersion weist ein hohes Aufnahmevermögen für derartige UV Filter auf. Die weitere Verarbeitung der so bereits vorgelöst vorliegenden UV Filter stellt dann kein problem mehr dar.

Bevorzugt enthält die ölige Dispersion daher vorzugsweise mindestens einen Filter aus der Gruppe der Dibenzoylmethan-Derivate, wobei insbesondere bevorzugt Methoxy-tert.-butyldibenzoylmethan enthalten ist, der benzophenon-Derivazte, wobei Benzophenone-3 bevorzugt enthalten ist, der Benzyldiden-Kampher-Derivate, wobei 4-Methylbenzylidene Camphor bevorzugt enthalten ist, Bis-Ethylhexyloxyphenol Methoxyphenyl Triazin, Ethylhexyl Triazon oder Diethylamino Hydroxybenzoyl Hexyl Benzoat.

Dabei kann die dispersion feste organische UV Filter in Mengen von 1 bis 20 Gew.-% bezogen auf die gesamte Dispersion, enthalten, wobei Gehalte von 5 bis 15 Gew.-% insbesondere bevorzugt sind.

Ein weiterer Gegenstand der vorliegenden Erfindung ist ein Verfahren zur Herstellung einer öligen Dispersion eines nanopartikulären UV-Schutzmittels, welches dadurch gekennzeichnet ist, dass ein Ester mit einem Dispergierhilfsmittel gemäß Anspruch 1 und einem pulverförmigen nanopartikulären UV-Schutzmittel mit Siliciumdioxid-Beschichtung vermischt wird.

Dabei kann das Mischen durch Rühren in einem üblichen Mischer oder durch Vermahlung in einem Naßmahlaggregat, z.B. mittels einer Perl- oder Kugelmühle, oder in einem Homogenisator erfolgen.

Vorzugsweise wird dabei eine Vormischung aus dem Ester und dem Dispergierhilfsmittel hergestellt und die Vormischung mit pulverförmigen nanopartikulären UV-Schutzmittel mit Siliciumdioxid-Beschichtung vermischt.

Wie bereits oben erwähnt, werden die öligen Dispersionen von nanopartikulären UV-Schutzmitteln mit den erfindungsgemäßen Eigenschaften beispielsweise erhalten, wenn für das nanopartikulären UV-Schutzmittel ein bestimmtes Herstellverfahren eingehalten wird. Die Hydrothermalbehandlung wird dabei vorzugsweise bei Temperaturen im Bereich von 40 bis 360˚C, vorzugsweise im Bereich von 80 bis 220˚C und insbesondere bevorzugt im Bereich von 140 bis 200˚C durchgeführt . Durch die Hydrothermalhehandlung kommt es zur Ausbildung stabiler Nano-Kristallite mit gleichmäßiger Größe und Form. Bei niedrigen Temperaturen entstehen "nadelförmige" Kristallite. Mit zunehmende Temperatur verrunden die Kristallite. Es bilden sich ovale Fomen, die bis zu runden Partikeln bei sehr hohen Temperaturen gehen. Zusätzlich kommt es zu einem gleichmäßigem Kristallwachstum, was zu einer Erniedrigung der Reaktivität und Photoaktivität führt. Vorteile der Hydrothermalbehandlung gegenüber einer üblichen thermischen Behandlung (Temperaturbehandlung eines getrockneten Pulvers) sind : Ausbildung gleichmäßiger Kristallitgrößen mit enger Kornverteilung und Verhinderung von Sintereffekten (Bildung von unerwünschten Aggregaten).

Die Siliciumdioxid-Beschichtung wird vorzugsweise als Sol-Gel-Prozess durchgeführt, wobei insbesondere bevorzugt eine Wasserglaslösung zu einer Suspension des Metalloxids gegeben wird. Dabei wird der Sol-Gel-Prozeß in einer vorteilhaften Variante der vorliegenden Erfindung bei konstant gehaltenem pH-Wert durchgeführt. Der konstant gehaltene pH-Wert kann in einem Bereich von pH 2 bis pH 11 liegen, wobei der pH-Wert vorzugsweise im Bereich von pH = 5 bis pH = 8, insbesonders bevorzugt im Bereich von pH = 6 bis pH = 7 liegt. Eine weitere vorteilhafte Verfahrensvariante ist die Gesamtzugabe des zur Nachbehandlung notwendigen Wasserglases bei einem pH = 7 bis pH = 11 ohne pH-Konstanthaltung. Anschließend wird der pH-Wert auf einen Wert von pH = 5 bis pH = 8, vorzugsweise auf pH = 6 bis pH = 7 abgesenkt. Weiter ist es bevorzugt, wenn die Beschichtung bei erhöhter Temperatur, vorzugsweise bei einer Temperatur im Bereich von 50˚C bis 110˚C durchgeführt wird.

Bei allen genannten Varianten des Verfahrens ist eine Reifezeit nach beendeter Beschichtung vorteilhaft. Die Reifezeit sollte zwischen 1 h und 8 h , bevorzugt 2 h bis 4 h betragen und bei einer Temperatur von 50˚C bis 110˚C durchgeführt werden.

Weiter kann es im Hinblick auf die bei der späteren Verarbeitung erwünschten Agglomeratgrößen von Vorteil sein, wenn das Produkt nachträglich vermahlen wird. Hier können die üblichen bei nanopartikulären Materialen verwendbaren Mahltechniken eingesetzt werden.

Aufgrund der oben genannten Vorteile ist ein weiterer Gegenstand der vorliegenden Erfindung eine Zubereitung mit Lichtschutzeigenschaften, welche mindestens eine erfindungsgemäße ölige Dispersion von nanopartikulärem UV-Schutzmittel enthält.

Bei den Zubereitungen handelt es sich in einer Erfindungsvariante vorzugsweise um topisch anwendbare Zubereitungen, beispielsweise kosmetische oder dermatologische Formulierungen. Die Zubereitungen enthalten in diesem Fall einen kosmetisch oder dermatologisch geeigneten Träger und je nach gewünschtem Eigenschaftsprofil optional weitere geeignete Inhaltsstoffe.

Weitere erfindungsgemäß bevorzugte Zubereitungen sind aus der Gruppe Fasern, Textilien, einschließlich deren Beschichtungen, Anstrichstoffe, Beschichtungssysteme, Folien und Verpackungen für den Schutz von Lebensmittel, Pflanzen oder technischen Gütern ausgewählt.

Ein weiterer Gegenstand der vorliegenden Erfindung ist dementsprechend die Verwendung einer erfindungsgemäßen öligen dispersion eines nanopartikulären UV-Schutzmittels oder eines nanopartikulären UV-Schutzmittel hergestellt nach einem erfindungsgemäßen Verfahren zur Einarbeitung in Anstrichstoffe, Beschichungssysteme, Folien, Verpackungen, Fasern, Textilien und Formteile aus Kautschuk oder Silikonkautschuk, wie Reifen oder Isolatoren.

Neben den bereits oben genannten Vorteilen kann der Einsatz der erfindungsgemäßen nanopartikulären UV-Schutzmittel in Zubereitungen, die Emulsionen sind, insbesondere auch zur Stabilisierung der Emulsion beitragen. Dadurch kann in der Regel der Einsatz von Emulgatoren verringert werden oder im Einzelfall (Pickering-Emulsion), sogar ganz auf den Einsatz von Emulgatoren verzichtet werden. Erfindungsgemäß bevorzugt sind daher auch emulgatorfreie Emulsionen, welche die erfindungegemäßen nanopartikulären UV-Schutzmittel enthalten.

Bevorzugte Zubereitungen mit Lichtschutzeigenschaften enthalten dabei mindestens ein Dibenzoylmethanderivat. Die im Rahmen der vorliegenden Erfindung verwendeten Dibenzoylmethanderivate sind, wie bereits gezeigt, an sich bereits wohlbekannte Produkte, die insbesondere in den oben genannten Druckschriften FR-A-2 326 405, FR-A-2 440 933 und EP-A-0 114 607 beschrieben sind.
Die erfindungsgemäß verwendbaren Dibenzoylmethanderivate können insbesondere unter den Dibenzoylmethanderivaten der folgenden Formel ausgewählt sein:

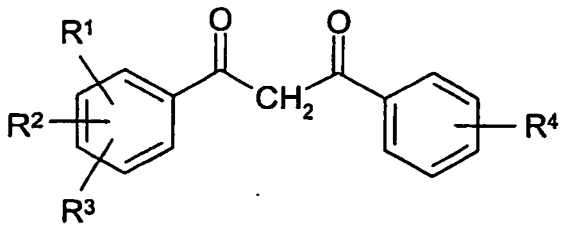

worin $R^1$, $R^2$, $R^3$ und $R^4$, die identisch oder voneinander verschieden sind, Wasserstoff, eine geradkettige oder verzweigte $C_{1-8}$-Alkylgruppe oder eine geradkettige oder verzweigte $C_{1-8}$-Alkoxygruppe bedeuten. Gemäß der vorliegenden Erfindung können selbstverständlich ein Dibenzoylmethanderivat oder mehrere Dibenzoylmethanderivate verwendet werden.
Von den Dibenzoylmethanderivaten, auf die sich die vorliegende Erfindung spezieller bezieht, können insbesondere:

- 2-Methyldibenzoylmethan,
- 4-Methyldibenzoylmethan,
- 4-Isopropyldigenzoylmethan,
- 4-tert.-Butyldibenzoylmethan,
- 2,4-Dimethyldibenzoylmethan,
- 2,5-Dimethyldibenzoylmethan,
- 4,4'-Diisopropyldibenzoylmethan,
- 4,4'-Methoxy-tert.-butyldibenzoylmethan,
- 2-Methyl-5-isopropyl-4'-methoxydibenzoylmethan,
- 2-Methyl-5-tert.-butyl-4'-methoxydibenzoylmethan,
- 2,4-Dimethyl-4'-methoxydibenzoylmethan und
- 2,6-Dimethyl-4-tert.-butyl-4'-methoxydibenzoylmethan genannt werden, wobei diese Aufzählung nicht einschränkend ist.
  Von den obengenannten Dibenzoylmethanderivaten wird erfindungsgemäß insbesondere das 4,4'-Methoxy-tert.-butyldibenzoylmethan und insbesondere das unter der Handelsbezeichnung Eusolex® 9020 von der Firma Merck im Handel befindliche 4,4'-Methoxy-tert.-butyldibenzoylmethan bevorzugt, wobei dieses Filter der folgenden Strukturformel entspricht:

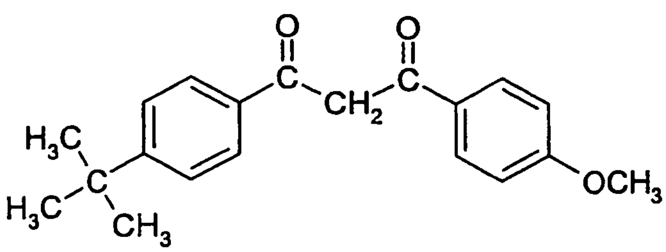

Ein weiteres erfindungsgemäß bevorzugtes Dibenzoylmethanderivat ist das 4-Isopropyldibenzoylmethan.
Weitere bevorzugte Zubereitungen mit Lichtschutzeigenschaften enthalten dabei mindestens ein Benzophenon oder Derivat des Benzophenon, wie insbesondere bevorzugt 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40).
Das oder die Dibenzoylmethanderivat(e) oder das oder die Benzophenonderivat(e) können in den erfindungsgemäßen Zusammensetzungen in Mengenanteilen vorliegen, die im Allgemeinen im Bereich von 0,1 bis 10 Gew.- % liegen und vorzugsweise in Mengenanteilen, die im Bereich von 0,3 bis 5 Gew.-% liegen, wobei diese Mengenanteile auf das Gesamtgewicht der Zusammensetzung bezogen sind.

Aufgrund der oben genannten Vorteile ist ein weiterer Gegenstand der vorliegenden Erfindung auch die Verwendung eines erfindungsgemäßen nanopartikulären Metalloxids mit Lichtschutzeigenschaften zur Stabilisierung von UV-Filtern, insbesondere Dibenzoylmethan und Derivaten des Dibenzoylmethan bzw. Benzophenon und Derivaten des Benzophenon.

Weiter kann es erfindungsgemäß bevorzugt sein, wenn die Zubereitungen weitere anorganische UV-Filter enthalten. Hierbei sind sowohl solche aus der Gruppe der Titandioxide wie z.B. gecoatetes Titandioxid (z.B. Eusolex® T-2000, Eusolex®TT-AQUA), Zinkoxide (z.B. Sachtotec®), Eisenoxide oder auch Ceroxide bevorzugt. Diese anorganischen UV-Filter werden in der Regel in einer Menge von 0,5 bis 20 Gewichtsprozent, vorzugsweise 2 - 10 %, in kosmetische Zubereitungen eingearbeitet. Insbesondere kann es dabei bevorzugt sein, wenn In Emulsionen in einer Phase ein erfindungsgemäßes nanopartikulkäres UV-Schutzmittel und in der anderen Phase ein weiterer anorganischer UV-Filter enthalten ist.

In einer weiteren ebenfalls bevorzugten Ausführungsform der vorliegenden Erfindung enthält die erfindungsgemäße Zubereitung mindestens einen Selbstbräuner.

Als vorteilhafte Selbstbräuner können unter anderem eingesetzt werden:

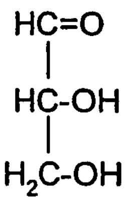

Glycerolaldehyd

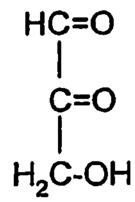

Hydroxymethylglyoxal

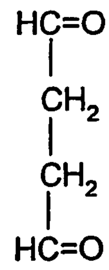

γ-Dialdehyd

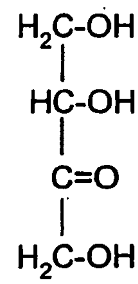

Erythrulose

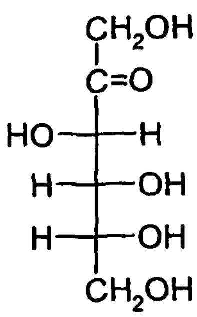

6-Aldo-D-Fructose

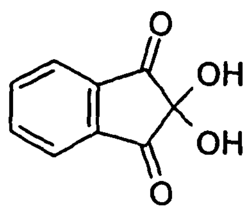

Ninhydrin

Ferner ist das 5-Hydroxy-1,4-naphtochinon (Juglon) zu nennen, das aus den Schalen frischer Walnüsse extrahiert wird

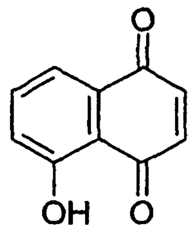

5-Hydroxy-1,4-naphtochinon (Juglon)

sowie das in den Henna-Blättern vorkommende 2-Hydroxy-1,4-naphtochinon (Lawson).

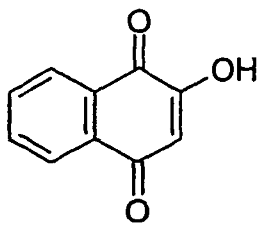

2-Hydroxy-1,4-naphtochinon (Lawson)

Ganz besonders bevorzugt ist das 1,3-Dihydroxyaceton (DHA), ein im menschlichen Körper vorkommender dreiwertiger Zucker und dessen Derivate.

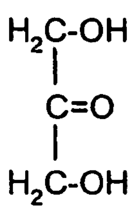

1,3-Dihydroxyaceton (DHA)

Die Verwendung einer erfindungsgemäßen öligen Dispersion eines nanopartikulären UV-Schutzmittels zur Stabilisierung von Selbstbräunern, insbesondere Dihydroxyaceton oder Dihydroxyacetonderivaten ist ein weiterer Gegenstand der vorliegenden Erfindung.

Ferner können die erfindungsgemäßen Zubereitungen auch Farbstoffe und Farbpigmente enthalten. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. $Fe_2O_3$, $Fe_3O_4$, FeO (OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

| Chemische oder sonstige Bezeichnung | CIN | Farbe |
|---|---|---|
| Pigment Green | 10006 | grün |
| Acid Green 1 | 10020 | Grün |

(fortgesetzt)

| Chemische oder sonstige Bezeichnung | CIN | Farbe |
|---|---|---|
| 2,4-Dinitrohydroxynaphthalin-7-sulfonsäure | 10316 | Gelb |
| Pigment Yellow 1 | 11680 | Gelb |
| Pigment Yellow 3 | 11710 | Gelb |
| Pigment Orange 1 | 11725 | Orange |
| 2,4-Dihydroxyazobenzol | 11920 | Orange |
| Solvent Red 3 | 12010 | Rot |
| 1-(2'-Chlor-4'-nitro-1'-phenylazo)-2-hydroxynaphthalin | 12085 | Rot |
| Pigment Red 3 | 12120 | Rot |
| Ceresrot; Sudanrot; Fettrot G | 12150 | Rot |
| Pigment Red 112 | 12370 | Rot |
| Pigment Red 7 | 12420 | Rot |
| Pigment Brown 1 | 12480 | Braun |
| 4-(2'-Methoxy-5'sulfonsäurediethylamid-1'-phenylazo)-3-hydroxy-5"-chloro-2",4"-dimethoxy2-naphthoesäureanilid | 12490 | Rot |
| Disperse Yellow 16 | 12700 | Gelb |
| 1-(4-Sulfo-1-phenylazo)-4-amino-benzol-5-sulfosäure | 13015 | Gelb |
| 2,4-Dihydroxy-azobenzol-4'-sulfosäure | 14270 | Orange |
| 2-(2,4-Dimethylphenylazo-5-sulfosäure)-1-hydroxynaphthalin-4-sulfosäure | 14700 | Rot |
| 2-(4-Sulfo-1-naphthylazo)-1-naphthol-4-sulfosäure | 14720 | Rot |
| 2-(6-Sulfo-2,4-xylylazo)-1-naphthol-5-sulfosäure | 14815 | Rot |
| 1-(4'-Sulfophenylazo)-2-hydroxynaphthalin | 15510 | Orange |
| 1-(2-Sulfosäure-4-chlor-5-carbonsäure-1-phenylazo)-2-hydroxynaphthalin | 15525 | Rot |
| 1-(3-Methyl-phenylazo-4-sutfosäure)-2-hydroxynaphthalin | 15580 | Rot |
| 1-(4',(8')-Sulfosäurenaphthylazo)-2-hydroxynaphthalin | 15620 | Rot |
| 2-Hydroxy-1,2'-azonaphthalin-1'-sulfosäure | 15630 | Rot |
| 3-Hydroxy-4-phenylazo-2-naphthylcarbonsäure | 15800 | Rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | Rot |
| 1-(2-Sulfo-1-naphthylazo)-2-hydroxynaphthalin-3-carbonsäure | 15880 | Rot |
| 1-(3-Sulfo-1-phenylazo)-2-naphthol-6-sulfosäure | 15980 | Orange |
| 1-(4-Sulfo-1-phenylazo)-2-naphthol-6-sulfosäure | 15985 | Gelb |
| Allura Red | 16035 | Rot |
| 1-(4-Sulfo-1-naphthylazo)-2-naphthol-3,6-disulfosäure | 16185 | Rot |
| Acid Orange 10 | 16230 | Orange |
| 1-(4-Sulfo-1-naphthylazo)-2-naphthol-6,8-disulfosäure | 16255 | Rot |
| 1-(4-Sulfo-1-naphthylazo)-2-naphthol-3,6,8-trisulfosäure | 16290 | Rot |
| 8-Amino-2-phenylazo-1-naphthol-3,6-disulfosäure | 17200 | Rot |
| Acid Red 1 | 18050 | Rot |
| Acid Red 155 | 18130 | Rot |
| Acid Yellow 121 | 18690 | Gelb |
| Acid Red 180 | 18736 | Rot |
| Acid Yellow 11 | 18820 | Gelb |
| Acid Yellow 17 | 18965 | Gelb |
| 4-(4-Sulfo-1-phenylazo)-1-(4-sulfophenyl)-5-hydroxy-pyrazolon-3-carbonsäure | 19140 | Gelb |
| Pigment Yellow 16 | 20040 | Gelb |
| 2,6-(4'-Sulfo-2",4"-dimethyl)-bis-phenylazo)1,3-dihydroxybenzol | 20170 | Orange |
| Acid Black 1 | 20470 | Schwarz |
| Pigment Yellow 13 | 21100 | Gelb |
| Pigment Yellow 83 | 21108 | Gelb |

(fortgesetzt)

| Chemische oder sonstige Bezeichnung | CIN | Farbe |
|---|---|---|
| Solvent Yellow | 21230 | Gelb |
| Acid Red 163 | 24790 | Rot |
| Acid Red 73 | 27290 | Rot |
| 2-[4'-(4"-Sulfo-1"-phenylazo)-7'-sulfo-1'-naphthylazo]-1-hydroxy-7-aminonaphthalin-3,6-disulfosäure | 27755 | schwarz |
| 4-[4"-Sulfo-1"-phenylazo)-7'-sulfo-1'-naphthylazo]-1-hydroxy-8-acetyl-aminonaphthalin-3,5-disulfosäure | 28440 | Schwarz |
| Direct Orange 34, 39, 44, 46, 60 | 40215 | Orange |
| Food Yellow | 40800 | Orange |
| trans-β-Apo-8'-Carotinaldehyd ($C_{30}$) | 40820 | Orange |
| trans-Apo-8'-Carotinsäure ($C_{30}$)-ethylester | 40850 | Orange |
| Canthaxanthin | 40850 | Orange |
| Acid Blue 1 | 42045 | Blau |
| 2,4-Disulfo-5-hydroxy-4'-4"-bis-(diethylamino)triphenyl-carbinol | 42051 | Blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | Grün |
| Acid Blue 7 | 42080 | Blau |
| (N-Ethyl-p-sulfobenzyl-amino)-phenyl-(2-sulfophenyl)-methylen-(N-ethyl-N-p-sulfo-benzyl) $\Delta^{2,5}$-cyclohexadienimin | 42090 | Blau |
| Acid Green 9 | 42100 | Grün |
| Diethyl-di-sulfobenzyl-di-4-amino-2-chlor-di-2-methyl-fuchsonimmonium | 42170 | Grün |
| Basic Violet 14 | 42510 | Violet |
| Basic Violet 2 | 42520 | Violet |
| 2'-Methyl-4'-(N-ethyl-N-m-sulfobenzyl)-amino-4"-(N-diethyl)-amino-2-methyl-N-ethylIN-m-sulfobenzyl-fuchsonimmonium | 42735 | Blau |
| 4'-(N-Dimethyl)-amino-4"-(N-phenyl)-aminonaphtho-N-dimethylfuchsonimmonium | 44045 | Blau |
| 2-Hydroxy-3,6-disulfo-4,4'-bis-dimethylaminonaphthofuchsonimmonium | 44090 | Grün |
| Acid Red 52 | 45100 | Rot |
| 3-(2'-Methylphenylamino)-6-(2'-methyl-4'-sulfophenylamino)-9-(2"-carboxyphenyl)-xantheniumsalz | 45190 | Violet |
| Acid Red 50 | 45220 | Rot |
| Phenyl-2-oxyfluoron-2-carbonsäure | 45350 | gelb |
| 4,5-Dibromfluorescein | 45370 | Orange |
| 2,4,5,7-Tetrabromfluorescein | 45380 | Rot |
| Solvent Dye | 45396 | Orange |
| Acid Red 98 | 45405 | Rot |
| 3',4',5',6'-Tetrachlor-2,4,5,7-tetrabromfluorescein | 45410 | Rot |
| 4,5-Diiodfluorescein | 45425 | Rot |
| 2,4,5,7-Tetraiodfluorescein | 45430 | Rot |
| Chinophthalon | 47000 | Gelb |
| Chinophthalon-disulfosäure | 47005 | Gelb |
| Acid Violet 50 | 50325 | Violett |
| Acid Black 2 | 50420 | Schwarz |
| Pigment Violet 23 | 51319 | Violett |
| 1,2-Dioxyanthrachinon, Calcium-Sluminiumkomplex | 58000 | Rot |
| 3-Oxypyren-5,8,10-sulfosäure | 59040 | Grün |
| 1-Hydroxy-4-N-phenyl-aminoanthrachinon | 60724 | Violett |
| 1-Hydroxy-4-(4'-methylphenylamino)-anthrachinon | 60725 | Violett |
| Acid Violet 23 | 60730 | Violett |

(fortgesetzt)

| Chemische oder sonstige Bezeichnung | CIN | Farbe |
|---|---|---|
| 1,4-Di(4'-methyl-phenylamino)-anthrachinon | 61565 | Grün |
| 1,4-Bis-(o-sulfo-p-toluidino)-anthrachinon | 61570 | Grün |
| Acid Blue 80 | 61585 | Blau |
| Acid Blue 62 | 62045 | Blau |
| N,N'-Dihydro-1,2,1',2'-anthrachinonazin | 69800 | Blau |
| Vat Blue 6; Pigment Blue 64 | 69825 | Blau |
| Vat Orange 7 | 71105 | orange |
| Indigo | 73000 | Blau |
| Indigo-disulfosäure | 73015 | Blau |
| 4,4'-Dimethyl-6,6'-dichlorthioindigo | 73360 | Rot |
| 5,5'Dichlor-7,T-dimethylthioindigo | 73385 | violett |
| Quinacridone Violet 19 | 73900 | violett |
| Pigment Red 122 | 73915 | Rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6, 19; Natural Red 1 | 75100 | gelb |
| Bixin, Nor-Bixin | 75120 | orange |
| Lycopin | 75125 | gelb |
| trans-alpha-, bet- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | Rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | Rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268 :1 | schwarz |
| Chromoxid | 77288 | grün |
| Chromoxid, wasserhaltig | 77278 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyahoferrat | 77510 | blau |
| Pigment White 18 | 77713 | weiß |

(fortgesetzt)

| Chemische oder sonstige Bezeichnung | CIN | Farbe |
|---|---|---|
| Mangananimoniumdiphosphat | 77742 | violett |
| Manganphosphat; $Mn_3(PO_4)_2 \cdot 7\ H_2O$ | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalzem, Anthocyane | | rot |
| Aluminium-, Zink-, Magnesium- und Calciumstearat | | weiß |
| Bromthymolblau | | blau |

Es kann ferner günstig sein, als Farbstoff eine oder mehrerer Substanzen aus der folgenden Gruppe zu wählen: 2,4-Dihydroxyazobenzol, 1-(2'-Chlor-4'-nitro-l'phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfonsäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfonsäure, Aluminiumsalz der 4-(4-Sulfo-1-phenylazo)-2-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77492), Manganammoniumdiphosphat und Titandioxid.

Ferner vorteilhaft sind öllösliche Naturfarbstoffe, wie z. B. Paprikaextrakt, β-Carotin oder Cochenille.

Vorteilhaft im Sinne der vorliegenden Erfindung sind ferner Gelcrèmes mit einem Gehalt an Perlglanzpigmenten. Bevorzugt sind insbesondere die im folgenden aufgelisteten Arten von Perlglanzpigmenten:

1. Natürliche Perlglanzpigmente, wie z. B.

- "Fischsilber" (Guanin/Hypoxanthin-Mischkristalle aus Fischschuppen) und
- "Perlmutt" (vermahlene Muschelschalen)

2. Monokristalline Perlglanzpigmente wie z. B. Bismuthoxychlorid (BiOCl)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteilhaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung/Schichtdicke | Farbe |
|---|---|---|
| **Silberweiße Perlglanzpigmente** | $TiO_2$: 40-60 nm | silber |
| **Interferenzpigmente** | $TiO_2$: 60-80 nm | gelb |
| | $TiO_2$: 80-100 nm | rot |
| | $TiO_2$: 100-140 nm | blau |
| | $TiO_2$: 120-160 nm | grün |
| **Farbglanzpigmente** | $Fe_2O_3$ | bronze |
| | $Fe_2O_3$ | kupfer |

(fortgesetzt)

| Gruppe | Belegung/Schichtdicke | Farbe |
|---|---|---|
| | $Fe_2O_3$ | rot |
| | $Fe_2O_3$ | rotviolett |
| | $Fe_2O_3$ | rotgrün |
| | $Fe_2O_3$ | schwarz |
| **Kombinationspigmente** | $TiO_2$ / $Fe_2O_3$ | Goldtöne |
| | $TiO_2$ / $Cr_2O_3$ | grün |
| | $TiO_2$ / Berliner Blau | tiefblau |

Besonders bevorzugt sind z. B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit $TiO_2$ und $Fe_2O_3$ beschichtete $SiO_2$-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Perlglanzpigmente, welche unter der Verwendung von $SiO_2$ hergestellt werden. Solche Pigmente , die auch zusätzlich gonichromatische Effekte haben können, sind z. B. unter dem Handelsnamen Sicopearl Fantastico bei der Firma BASF erhältlich.

Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks erhältlich. Sie weisen durch ihre Partikelgröße von 40-80 $\mu$m zusätzlich zu der Farbe einen Glitzereffekt auf.

Besonders vorteilhaft sind ferner auch Effektpigmente, welche unter der Handelsbezeichnung Metasomes Standard / Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.% bis 30 Gew.%, vorzugsweise von 0,5 bis 15 Gew.%, insbesondere von 1,0 bis 10 Gew.% gewählt, jeweils bezogen auf das Gesamtgewicht der Zubereitungen.

Erfindungsgemäß können die nanopartikulären UV-Schutzmittel auch mit einer die hydrophilen oder die hydrophoben Eigenschaften verstärkenden Oberflächenbehandlung versehen sein. Zur hydropoben Modifikation eignet sich beispielsweise eine Silicon- oder Silan-Beschichtung

Die Silicone sind bekanntlich silicium-organische Polymere oder Oligomere mit geradkettiger oder cyclischer, verzweigter oder vernetzter Struktur mit unterschiedlichen Molekülgewichten, die durch Polymerisation und/oder Polykondensation geeignet funktionalisierter Silane erhalten werden und im Wesentlichen aus wiederkehrenden Haupteinheiten gebildet werden, in denen die Siliciumatome über Sauerstoffatome miteinander verknüpft sind (Siloxanbindung), wobei gegebenfalls substituierte Kohlenwasserstoffgruppen über ein Kohlenstoffatom direkt an die Siliciumatome gebunden sind. Die gebräulichsten Kohlenwasserstoffgruppen sind die Alkylgruppen und inbesondere Methyl, die Fluoralkylgruppen, die Arylgruppen und insbesondere Phenyl sowie die Alkenylgruppen und insbesondere Vinyl. Weitere Typen von Gruppen, die entweder direkt oder über eine Kohlenwasserstoffgruppe an die Siloxankette gebunden werden können, sind insbesondere Wasserstoff, die Halogene und insbesondere Chlor, Brom oder Fluor, die Thiole, die Alkoxygruppen, die Polyoxyalkylengruppen (oder Polyether) und insbesondere Polyoxyethylen und/oder Polyoxypropylen, Hydroxygruppen oder Hydroxyalkylgruppen, die gegebenenfalls substituierten Aminogruppen, die Amidgruppen, die Acyloxygruppen oder Acyloxyalkylgruppen, die Hydroxyalkylaminogruppen oder Aminoalkylgruppen, quaternäre Ammoniumgruppen, amphotere Gruppen oder Betaingruppen, anionische Gruppen, wie Carboxylate, Thioglykolate, Sulfosuccinate, Thiosulfate, Phosphate und Sulfate, wobei diese Aufzählung selbstverständlich in keiner Weise einschränkend ist (sogenannte 'organomodiflzierte' Silicone).

Im Sinne der vorliegenden Erfindung sollen mit dem Ausdruck 'Silicone' auch die zu ihrer Herstellung benötigten Silane und insbesondere die Alkylsilane eingeschlossen und abgedeckt sein.

Die für die vorliegende Erfindung geeigneten Silicone, die zum Umhüllen der nanopartikulären UV-Schutzmittel verwendet werden können, sind vorzugsweise unter den Alkylsilanen, den Polydialkylsiloxanen und den Polyalkylhydrogensiloxanen ausgewählt. Noch bevorzugter sind die Silicone unter Octyltrimethylsilan, den Polydimethylsiloxanen und den Polymethylhydrogenosiloxanen ausgewählt.

Die nanopartikulären UV-Schutzmittel können in den erfindungsgemäßen Zusammensetzungen in Mengenanteilen vorliegen, die im allgemeinen im Bereich von 0,1 bis 50 Gew.-% liegen und vorzugsweise in Mengenanteilen, die im Bereich von 0,5 bis 20 Gew.-% liegen, wobei diese Mengenanteile auf das Gesamtgewicht der Zusammensetzung bezogen sind.

Die erfindungsgemäßen Sonnenschutzmittel können selbstverständlich einen oder mehrere zusätzliche(n) hydrophile(n) oder lipophile(n) Sonnenschutzfilter, die im UV-A-Bereich und/oder UV-B-Bereich und(/oder IR und/oder VIS-Bereich (Absorber) wirksam sind, enthalten,. Diese zusätzlichen Filter können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische Filter sind in der Patentanmeldung EP-A 0 487 404 angegeben.

Prinzipiell kommen alle UV-Filter für eine Kombination mit den erfindungsgemäßen nanopartikuläre UV-Schutzmitteln in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B.

Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxobom-3-yliden)toluol-4-sulfonsäure (z.B. Mexoryl® SL),

Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000),

Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS),

4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25),

Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure;
und weitere Substanzen wie

- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2-ethylhexyl-1-oxi)-1,3,5-triazin ( z.B. Uvinul® T 150)
- 2-(4-Diethylamino-2-hydroxy-benzoyl)-benzoesäure hexylester (z.B. Uvinul®UVA Plus, Fa. BASF).

Die in der Liste aufgeführten Verbindungen sind nur als Beispiele aufzufassen. Selbstverständlich können auch andere UV-Filter verwendet werden. Insbesondere können vorteilhaft auch organische partikuläre UV-Filter, wie Sie beispielsweise in der Patentanmeldung WO 99/66896 beschrieben sind, mit den erfindunsgsgemäßen nanopartikulären UV-Schutzmitteln kombiniert werden.

Diese organischen UV-Filter werden in der Regel in einer Menge von 0,5 bis 20 Gewichtsprozent, vorzugsweise 1 - 10 Gew.-%, in kosmetische Formulierungen eingearbeitet.

Weitere geeignete organische UV-Filter sind z.B.

- 2-(2H-Benzotriazol-2-yl)4-methyl-6-(2-methyl-3-(1,3,3,3-tetramethyl-1-(trimethylsilyloxy)disiloxanyl)propyl)phenol (z.B. Silatrizole®),
- 4,4'-[(6-[4-((1,1-Dimethylethyl)aminocarbonyl)phenylamino]-1,3,5-triazin-2,4-diyl)dümino]bis(benzoesäure-2-ethylhexylester) (z.B. Uvasorb® HEB),
- α-(Trimethylsityl)-ω-[trimethylsilyl)oxy]poly[oxy(dimethyl [und ca. 6% methyl[2-[p-[2,2-bis(ethoxycarbonyl]vinyl] phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1)
- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).

- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),

Organische UV-Filter werden insgesamt in der Regel in einer Menge von 0,5 bis 20 Gewichtsprozent, vorzugsweise 1-15 %, in kosmetische Formulierungen eingearbeitet.

Bevorzugte Verbindungen mit UV-filtemden Eigenschaften sind 3-(4'-Methylbenzyliden)-dl-kampfer, 1-(4-tert-Butylphenyl)-3-(4-methoxy-phenyl)-propan-1,3-dion, 4-Isopropyldibenzoylmethan, 2-Hydroxy-4-meth-oxy-benzophenon, Methoxyzimtsäureoctylester, 3,3,5-Trimethyl-cyclo-hexyl-sali-cylat, 4-(Dimethylamino)benzoesäure-2-ethyl-hexylester, 2-Cyano-3,3-di-phenyl-acrylsäure-2-ethylhexylester, 2-Phenyl-benzimidazol-5-sulfon-säure sowie ihre Kalium-, Natrium- und Triethanolaminsalze.

In einer bevorzugten Variante der erfindungsgemäßen Zubereitungen werden die bei 25°C festen öllöslichen organischen UV Filter zumindest teilweise, wie oben beschrieben, über die ölige Dispersion in die Lichtschutzzubereitungen eingebracht.

Bevorzugte Zubereitungen können auch Verbindungen der Formel I enthalten,

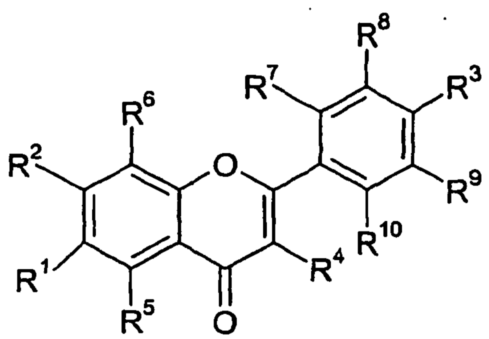

I

wobei $R^1$ und $R^2$ ausgewählt sind aus

- H
- und $OR^{11}$, wobei $OR^{11}$ unabhängig voneinander steht für
  - OH
  - geradkettige oder verzweigte $C_1$- bis $C_{20}$-Alkyloxygruppen,
  - geradkettigen oder verzweigten $C_3$- bis $C_{20}$-Alkenyloxygruppen,
  - geradkettigen oder verzweigten $C_1$- bis $C_{20}$-Hydroxyalkoxy-gruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder
  - $C_3$- bis $C_{10}$-Cycloalkyloxygruppen und/oder $C_3$- bis $C_{12}$-Cycloalkenytoxygruppen, wobei die Ringe jeweils auch durch - $(CH_2)_n$-Gruppen mit n = 1 bis 3 überbrückt sein können und/oder,
  - Mono- und/oder Oligoglycosylreste,

  mit der Maßgabe, dass mindestens ein Rest aus $R^1$ und $R^2$ steht für $OR^{11}$, und $R^3$ steht für einen Rest $OR^{11}$ und $R^4$ bis $R^7$ und $R^{10}$ gleich oder verschieden sein können, und unabhängig voneinander stehen für
- H
- geradkettige oder verzweigte $C_1$- bis $C_{20}$-Alkylgruppen,
- geradkettige oder verzweigte $C_3$- bis $C_{20}$-Alkenylgruppen,
- geradkettige oder verzweigte $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können und

  $R^8$ und $R^9$ gleich oder verschieden sein können, und unabhängig voneinander stehen für
- H
- $OR^{11}$
- geradkettige oder verzweigte $C_1$- bis $C_{20}$-Alkylgruppen,

- geradkettige oder verzweigte $C_3$- bis $C_{20}$-Alkenylgruppen,
- geradkettige oder verzweigte $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können.

Vorteile der erfindungsgemäßen Zusammensetzungen sind dabei insbesondere die UV-Licht filternde Wirkung und die gute Hautverträglichkeit. Zusätzlich sind die hier beschriebenen Verbindungen der Formel I farblos oder nur schwach gefärbt und führen so, im Gegensatz zu vielen bekannten natürlich vorkommenden Flavonoiden, nicht zu Verfärbungen der Zubereitungen.

Unter den erfindungsgemäß einzusetzenden Flavonoiden der Formel I finden sich dabei Breitband-UV-Filter, andere ebenfalls bevorzugte Verbindungen der Formel I zeigen ein Absorptionsmaximum im Grenzbereich zwischen der UV-B- und der UV-A-Strahlung. Als UV-A-II-Filter ergänzen sie daher vorteilhaft das Absorptionsspektrum von handelsüblichen UV-B- bzw. UV-A-I-Filtern. Bevorzugte erfindungsgemäße Zubereitungen mit Lichtschutzeigenschaften enthalten zumindest eine Verbindung der Formel I, wobei $R^3$ steht für

- OH oder
- geradkettige oder verzweigte $C_1$- bis $C_{20}$-Alkoxygruppen, vorzugsweise Methoxy, Ethoxy oder Ethylhexyloxy oder
- Mono- und/oder Oligoglycosylreste, vorzugsweise Glucosylreste und $R^1$ und/oder $R^2$ vorzugsweise stehen für
- OH oder
- geradkettige oder verzweigte $C_1$- bis $C_{20}$-Alkoxygruppen, vorzugsweise Methoxy, Ethoxy oder Ethylhexyloxy oder
- Mono- und/oder Oligoglycosylreste, vorzugsweise Glucosylreste.

Diese bevorzugten Verbindungen zeichnen sich durch eine besonders intensive UV-Absorption aus.

Zusätzlich haben solche bevorzugten Verbindungen Vorteile bei der Einarbeitung in die Zubereitungen:

- Mono- und/oder Oligoglycosylreste verbessern die Wasserlöslichkeit der erfindungsgemäß einzusetzenden Verbindungen;
- geradkettige oder verzweigte $C_1$- bis $C_{20}$-Alkoxygruppen, insbesondere die langkettigen Alkoxyfunktionen, wie Ethylhexyloxy-Gruppen erhöhen die Öllöslichkeit der Verbindungen;

d.h. über die geeignete Auswahl der Substituenten kann die Hydrophilie bzw. Lipophilie der Verbindungen nach Formel I gesteuert werden. Als Mono- oder Oligosaccharidreste bevorzugt sind dabei Hexosylreste, insbesondere Ramnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch vorteilhaft sein, Pentosylreste zu verwenden. Die Glycosylreste können $\alpha$- oder $\beta$-glycosidisch mit dem Grundkörper verbunden sein. Ein bevorzugtes Disaccharid ist beispielsweise das 6-O-(6-deoxy-a-L-mannopyranosyl)-$\beta$-D-glucopyranosid.

Es hat sich gezeigt, dass die Intensität der UV-Absorption insbesondere dann hoch ist, wenn $R^3$ steht für geradkettige oder verzweigte $C_1$- bis $C_{20}$-Alkoxygruppen, vorzugsweise Methoxy, Ethoxy oder Ethylhexyloxy, und $R^8$ und $R^9$ gleich sind und stehen für H oder geradkettige oder verzweigte $C_1$- bis $C_{20}$-Alkoxygruppen, vorzugsweise Methoxy, Ethoxy oder Ethylhexyloxy.
Daher sind Zubereitungen mit Lichtschutzeigenschaften enthaltend zumindest eine Verbindung der Formel I, die dadurch gekennzeichnet ist, dass $R^3$ steht für geradkettige oder verzweigte $C_1$- bis $C_{20}$-Alkoxygruppen, vorzugsweise Methoxy, Ethoxy oder Ethylhexyloxy, und $R^8$ und $R^9$ gleich sind und stehen für H oder geradkettige oder verzweigte $C_1$- bis $C_{20}$-Alkoxygruppen, vorzugsweise Methoxy, Ethoxy oder Ethylhexyloxy, erfindungsgemäß besonders bevorzugt. Insbesondere ist es dabei bevorzugt, wenn und $R^8$ und $R^9$ für H stehen.

Die Verbindungen der Formel I werden erfindungsgemäß typisch in Mengen von 0,01 bis 20 Gew.-%, vorzugsweise in Mengen von 0,5 Gew.-% bis 10 Gew.-% und insbesondere bevorzugt in Mengen von 1 bis 8 Gew.-% eingesetzt. Dabei bereitet es dem Fachmann keinerlei Schwierigkeiten die Mengen abhängig von dem beabsichtigten Lichtschutzfaktor der Zubereitung entsprechend auszuwählen.

Durch Kombination von einer oder mehrerer nanopartikulärer UV-Schutzmittel mit weiteren UV-Filtern kann die Schutzwirkung gegen schädliche Einwirkungen der UV-Strahlung optimiert werden. Optimierte Zusammensetzungen können beispielsweise die Kombination der organischen UV-Filter 4'-Methoxy-6-hydroxyflavon mit 1-(4-tert-Butylphenyl)-3-(4-methoxyphenyl)propan-1,3-dion und 3-(4'-Methylbenzyliden)-dl-kampfer enthalten.

Alle genannten UV-Filter einschließlich der Verbindungen der Formel I können auch in verkapselter Form eingesetzt werden. Insbesondere ist es von Vorteil organische UV-Filter in verkapselter Form einzusetzen. Im Einzelnen ergeben sich die folgende Vorteile:

- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.

- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.

- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.

- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher kann es erfindungsgemäß bevorzugt sein, wenn ein oder mehrere der Verbindungen gemäß Formel I bzw. der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Erfindungsgemäß besonders bevorzugt einzusetzende Kapseln weisen Wände auf, die durch einen SolGel-Prozeß, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Siliciumoxidhydroxid) aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört.

Dabei sind die Kapseln in erfindungsgemäßen Zubereitungen vorzugsweise in solchen Mengen enthalten, die gewährleisten, dass die verkapselten UV-Filter in den oben angegebenen Mengen in der Zubereitung vorliegen.

Weisen die erfindungsgemäßen Zubereitungen Verbindungen entsprechend Formel I mit freien Hydroxy-Gruppen auf, so zeigen sie neben den beschriebenen Eigenschaften zusätzlich eine Wirkung als Antioxidans und/oder Radikalfänger Bevorzugt sind daher auch Zubereitungen mit Lichtschutzeigenschaften enthaltend zumindest eine Verbindung der Formel I, die dadurch gekennzeichnet ist, dass mindestens einer der Reste $R^1$ bis $R^3$ steht für OH, wobei vorzugsweise mindestens einer der Reste $R^1$ oder $R^2$ für OH steht.

Damit die Verbindungen der Formel I ihre positive Wirkung als Radikalfänger auf die Haut besonders gut entwickeln können, kann es bevorzugt sein die Verbindungen der Formel I in tiefere Hautschichten eindringen zu lassen. Dazu stehen mehrere Möglichkeiten zur Verfügung. Zum einen können die Verbindungen der Formel I eine ausreichende Lipophilie aufweisen, um durch die äußere Hautschicht in epidermale Schichten vordringen zu können. Als weitere Möglichkeit können in der Zubereitung auch entsprechende Transportmittel, beispielsweise Liposomen, vorgesehen sein, die einen Transport der Verbindungen der Formel I durch die äußeren Hautschichten ermöglichen. Schließlich ist auch ein systemischer Transport der Verbindungen der Formel I denkbar. Die Zubereitung wird dann beispielsweise so gestaltet, dass sie für eine orale Gabe geeignet ist.

Allgemein wirken die Substanzen der Formel I als Radikalfänger. Solche Radikale werden nicht nur durch Sonnenlicht erzeugt, sondern werden unter verschiedenen Bedingungen gebildet. Beispiele sind Anoxie, die den Elektronenfluß stromauf der Cytochromoxidasen blockiert und die Bildung von Superoxidradikalarionen bedingt; Entzündungen, die unter anderem mit der Bildung von Superoxidanionen durch die Membran-NADPH-Oxidase der Leukozyten einhergehen, die jedoch auch mit der Bildung (durch Disproportionierung in Gegenwart von Eisen (II)-ionen) der Hydroxyradikale und anderer reaktiver Spezies, die normalerweise beim Phänomen einer Phagocytose beteiligt sind, einhergehen; sowie Lipidautooxidation die im Allgemeinen durch ein Hydroxylradikal initiiert wird und lipidische Alkoxyradikale und Hydroperoxide liefert.

Es wird vermutet, dass bevorzugte Verbindungen der Formel I auch als Enzymhemmer wirken. Sie hemmen vermutlich Histidindecarboxylase, Proteinkinasen, Elastase, Aldosereduktase sowie Hyaluronidase, und ermöglichen daher, die Unversehrtheit der Grundsubstanz vaskulärer Hüllen aufrecht zu erhalten. Ferner hemmen sie vermutlich nicht spezifisch Katechol-O-methyltransferase, wodurch die Menge der verfügbaren Katecholamine und dadurch die Gefäßfestigkeit

erhöht wird. Weiter hemmen sie AMP-Phosphodiesterase, wodurch die Substanzen ein Potential zur Hemmung der Thrombozytenaggregation aufweisen.
Aufgrund dieser Eigenschaften eignen sich die erfindungsgemäßen Zubereitungen allgemein zur Immunprotektion und zum Schutz der DNA und RNA. Insbesondere eignen sich die Zubereitungen dabei zum Schutz von DNA und RNA vor oxidativen Angriffen, vor Radikalen und vor Schädigung durch Strahlung, insbesondere UV-Strahlung. Ein weiterer Vorteil der erfindungsgemäßen Zubereitungen ist der Zellschutz, insbesondere der Schutz von Langerhans-Zellen vor Schäden durch die oben genannten Einflüsse. Alle diese Verwendungen bzw. die Verwendung der Verbindungen der Formel I zur Herstellung entsprechend einsetzbarer Zubereitungen sind ausdrücklich auch Gegenstand der vorliegenden Erfindung.

Insbesondere eignen sich bevorzugte erfindungsgemäße Zusammensetzungen auch zur Behandlung von Hautkrankheiten, die mit einer Störung der Keratinisierung verbunden sind, die die Differenzierung und Zellproliferation betrifft, insbesondere zur Behandlung der Akne vulgaris, Akne comedonicä, der polymorphen Akne, der Akne rosaceae, der nodulären Akne, der Akne conglobata, der alters-bedingten Aknen, der als Nebenwirkung auftretenden Aknen, wie der Akne solaris, der medikamenten-bedingten Akne oder der Akne professionalis, zur Behandlung anderer Störungen der Keratinisierung, insbesondere der Ichtyosen, der ichtyosiformen Zustände, der Darrier-Krankheit, der Keratosis palmoplantaris, der Leukoplasien, der leukoplasiformen Zustände, der Haut- und Schleimhautflechten (Buccal) (Lichen), zur Behandlung anderer Hauterkrankungen, die mit einer Störung der Keratinisierung zusammenhängen und eine entzündliche und/oder immunoallergische Komponente haben und insbesondere aller Formen der Psoriasis, die die Haut, die Schleimhäute und die Finger und Zehennägel betreffen, und des psoriatischen Rheumas und der Hautatopien, wie Ekzemen oder der respiratorischen Atopie oder auch der Hypertrophie des Zahnfleisches, wobei die Verbindungen ferner bei einigen Entzündungen verwendet werden können, die nicht mit einer Störung der Keratinisierung zusammenhängen, zur Behandlung aller gutartigen oder bösartigen Wucherungen der Dermis oder Epidermis, die gegebenenfalls viralen Ursprungs sind, wie Verruca vulgaris. Veruca plana, Epidermodysplasia verruciformis, orale Papillomatose, Papillomatosis florida, und der Wucherungen, die durch UV-Strahlung hervorgerufen werden können, insbesondere des Epithelioma baso-cellulare und Epithelioma spinocellulare, zur Behandlung anderer Hautkrankheiten, wie der Dermatitis bullosa und der das Kollagen betreffenden Krankheiten, zur Behandlung bestimmter Augenkrankheiten, insbesondere der Homhauterkrankungen, zur Behebung oder Bekämpfung der lichtbedingten und der mit dem Älterwerden zusammenhängenden Hautalterung, zur Verminderung der Pigmentierungen und der Keratosis actinica und zur Behandlung aller Krankheiten, die mit der normalen Alterung oder der lichtbedingten Alterung zusammenhängen, zur Vorbeugung vor oder der Heilung von Wunden/Narben der Atrophien der Epidermis und/oder Dermis, die durch lokal oder systemisch angewendete Corticosteroide hervorgerufen werden und aller sonstigen Arten der Hautatrophie, zur Vorbeugung vor oder Behandlung von Störungen der Wundheilung, zur Vermeidung oder Behebung von Schwangerschaftsstreifen oder auch zur Förderung der Wundheilung, zur Bekämpfung von Störungen der Talgproduktion, wie Hypersebhorrhö bei Akne oder der einfachen Seborrhö, zur Bekämpfung von oder Vorbeugung von krebsartigen Zuständen oder vor präkanzerogenen Zuständen, insbesondere der promyelozytären Leukämien, zur Behandlung von Entzündungserkrankungen, wie Arthritis, zur Behandlung aller virusbedingten Erkrankungen der Haut oder anderer Bereiche des Körpers, zur Vorbeugung vor oder Behandlung der Alopecie, zur Behandlung von Hautkrankheiten oder Krankheiten anderer Körperbereiche mit einer immunologischen Komponente, zur Behandlung von Herz- /Kreislauf-Erkran-kungen, wie Arteriosklerose oder Bluthochdruck, sowie des Insulin-unabhängigen Diabetes, zur Behandlung von Hautproblemen, die durch UV-Strahlung hervorgerufen werden.

Die schützende Wirkung gegen oxidativen Stress bzw. gegen die Einwirkung von Radikalen kann weiter verbessert werden, wenn die Zubereitungen ein oder mehrere Antioxidantien enthalten.

In einer bevorzugten Ausführungsform der vorliegenden Erfindungen handelt es sich bei der Zubereitung daher um eine Zubereitung zum Schutz von Körperzellen gegen oxidativen Stress, insbesondere zur Verringerung der Hautalterung, **dadurch gekennzeichnet, dass** sie vorzugsweise ein oder mehrere Antioxidantien enthält.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Camosin, D-Carnosin, L-Camosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsuifoximin, Buthioninsulfone, Penta-, Hexa-, Heptathioninsulfoximin) in sehr geringen verträglichen Dosierungen (z.B. pmol bis $\mu$mol/kg), ferner (Metall-) Chelatoren, (z.B. $\alpha$-Hydroxyfettsäuren, Palmitinsäure, Phytinsäure, Lactoferrin), $\alpha$-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordohydroguajaretsäure, Tri-hydroxybutyrophenon, Quercitin, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B.

Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004).

Die erfindungsgemäßen Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-α-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Zubereitungen enthalten, insbesondere bevorzugt Vitamin-A-Palmitat, Vitamin C, DL-α-Tocopherol, Tocopherol-E-Acetat, Nicotinsäure, Pantothensäure und Biotin.

Die erfindungsgemäßen Zubereitungen können darüber hinaus weitere übliche hautschonende oder hautpflegende Wirkstoffe enthalten. Dies können prinzipiell alle den Fachmann bekannten Wirkstoffe sein.

Besonders bevorzugte Wirkstoffe sind Pyrimidincarbonsäuren und/oder Aryloxime.

Pyrimidincarbonsäuren kommen in halophilen Mikroorganismen vor und spielen bei der Osmoregulation dieser Organismen eine Rolle (E. A. Galinski et al., Eur. J. Biochem., 149 (1985) Seite 135-139). Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Pyrimidincarbonsäure gemäß der unten stehenden Formel II eingesetzt,

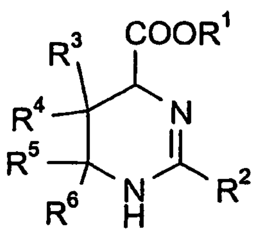

II

worin $R^1$ ein Rest H oder C1-8-Alkyl, $R^2$ ein Rest H oder C1-4-Alkyl und $R^3$, $R^4$, $R^5$ sowie $R^8$ jeweils unabhängig voneinander ein Rest aus der Gruppe H, OH, $NH_2$ und C1-4-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind.

Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäßen Zubereitungen derartige Pyrimidincarbonsäuren vorzugsweise in Mengen bis zu 15 Gew.-%.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die Aryloxime, vorzugsweise 2-Hydroxy-5-methyllaurophenonoxim enthalten, zeigen überraschende antiinflammatorische Eignung. Dabei enthalten die Zubereitungen vorzugsweise 0,01 bis 10 Gew.-% des Aryloxims, wobei es insbesondere bevorzugt ist, wenn die Zubereitung 0,05 bis 5 Gew-% Aryloxim enthält.

Alle hier beschriebenen Verbindungen oder Komponenten, die in den Zubereitungen verwendet werden können, sind entweder bekannt und käuflich erwerbbar oder können nach bekannten Verfahren synthetisiert werden.

Neben den hier beschriebenen Verbindungen können die erfindungsgemäßen Zubereitungen auch mindestens einen Photostabilisator, vorzugsweise entsprechend der Formel III

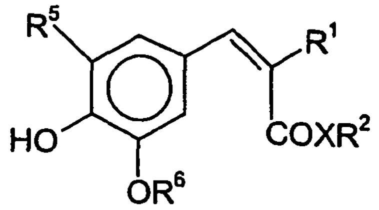

III,

wobei

$R^1$ ausgewählt ist aus -C(O)$CH_3$, -$CO_2R^3$, -C(O)$NH_2$ and -C(O)N$(R^4)_2$;
X is O or NH;
$R^2$ steht für einen linearen oder verzweigten $C_{1-30}$-Alkylrest;
$R^3$ steht für einen linearen oder verzweigten $C_{1-20}$-Alkylrest, alle $R^4$ unabhängig voneinander stehen für H oder lineare oder verzweigte $C_{1-8}$-Alkylreste
$R^5$ steht für H, einen linearen oder verzweigten $C_{1-8}$-Alkylrest oder einen linearen oder verzweigten -O-$C_{1-8}$-Alkylrest und
$R^6$ steht für einen $C_{1-8}$-Alkylrest,

wobei es sich bei dem Photostabilisator insbesondere bevorzugt um 2-(4-Hydroxy-3,5-dimethoxy-benzyliden)-malonsäure-bis-(2-ethyl-hexyl)ester handelt, enthalten. Entsprechende Photostabilisatoren, ihre Herstellung und Verwendung sind in der Internationalen Patentanmeldung WO 03/007906 beschrieben, deren Offenbarung ausdrücklich auch zum Gegenstand der vorliegenden Anmeldung gehört.

Die erfindungsgemäßen Zusammensetzungen können nach Verfahren hergestellt werden, die dem Fachmann gut bekannt sind, insbesondere nach den Verfahren, die zur Herstellung von Öl-in-Wasser-Emulsionen oder Wasser-in-Öl-Emulsionen dienen.

Weitere Gegenstände der vorliegenden Erfindung sind ein Verfahren zur Herstellung einer Zubereitung gemäß Anspruch 18 welches dadurch gekennzeichnet ist, dass mindestens eine ölige Dispersion eines nanopartikuläres UV-Schutzmittels mit einem kosmetisch oder dermatologisch geeignetem Träger und ggf. weiteren Inhaltstoffen vermischt wird und die Verwendung von öligen Dispersionen nanopartikulärer UV-Schutzmittel zur Herstellung einer Zubereitung mit Lichtschutzeigenschaften gemäß Anspruch 19.

Diese Zusammensetzungen können insbesondere in Form von einfachen oder komplizierten Emulsionen (O/W, W/O, O/W/O oder W/O/W), wie Cremes, Milchen, Gelen oder Gel-Cremes, Pulvern und festen Stiften, vorliegen und gegebenenfalls können sie als Aerosole konfektioniert sein und in Form von Schäumen oder Sprays vorliegen. Vorzugsweise liegen diese Zusammensetzungen in Form einer O/W-Emulsion vor.

Die erfindungsgemäßen kosmetischen Zusammensetzungen können als Zusammensetzungen zum Schutz der menschlichen Epidermis oder der Haare gegen UV-Strahlung, als Sonnenschutzmittel oder Schminkprodukte verwendet werden.

Es soll darauf hingewiesen werden, dass in den erfindungsgemäßen Formulierungen zum Sonnenschutz, die

einen Träger vom Typ einer Öl-in-Wasser-Emulsion aufweisen, die wässerige Phase (die insbesondere die hydrophilen Filter enthält) im Allgemeinen 50 bis 95 Gew.-% und vorzugsweise 70 bis 90 Gew.-%, bezogen auf die gesamte Formulierung, die Ölphase (die insbesondere die lipophilen Filter enthält) 5 bis 50 Gew.-% und vorzugsweise 10 bis 30 Gew.- %, bezogen auf die gesamte Formulierung und der (Co)emulgator oder die (Co)emulgatoren 0,5 bis 20 Gew.-% und vorzugsweise 2 bis 10 Gew.-%, bezogen auf die gesamte Formulierung, ausmachen.

Geeignet sind Zubereitungen für eine äußerliche Anwendung, beispielsweise als Creme, Lotion, Gel, oder als Lösung, die auf die Haut aufgesprüht werden kann. Für eine innerliche Anwendung sind Darreichungsformeln wie Kapseln, Dragees, Pulver, Tabletten-Lösungen oder Lösungen geeignet.

Als Anwendungsform der erfindungsgemäßen Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestem, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestem, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Zu den bevorzugten erfindungsgemäßen Zubereitungsformen gehören insbesondere Emulsionen.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettlkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung kann gewählt werden aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.
Insbesondere bevorzugt enthält die Ölphase jedoch die auch für die erfindungsgemäße Dispersion einzusetzenden Ester von gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkylenglykolen einer Kettenlänge von 2 bis 30 C-Atomen als wesentlichen Bestandteil. Wie bereits weiter oben bescjhrieben ist dabei insbesondere Butylenglykoldicaprylat/dicaprat als Öl bevorzugt.
Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als Lipidkomponente der Ölphase einzusetzen.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearan Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemisch der vorstehend genannten Lösemittel verwendet. Bei alkoholischen Lösemitteln kann Wasser ein weiterer Bestandteil sein.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Formuierung verwendet wird.

In einer bevorzugten Ausführungsform enthalten die erfindungsgemäßen Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Die Alkylglucoside werden ihrerseits vorteilhaft gewählt aus der Gruppe der Alkylglucoside, welche sich durch die Strukturformel

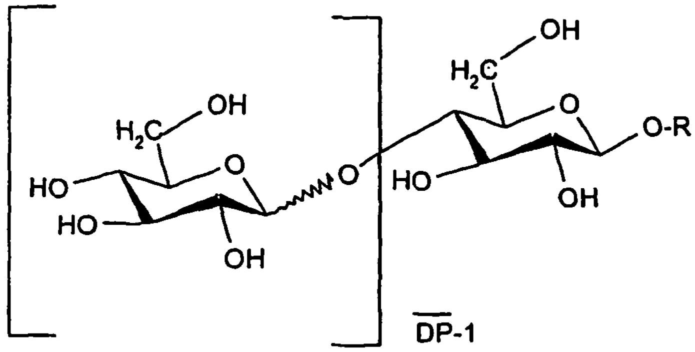

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet.

Der Wert $\overline{DP}$ repräsentiert den Glucosidierungsgrad der erfindungsgemäß verwendeten Alkylglucoside und ist definiert als

$$\overline{DP} = \frac{p_1}{100} \cdot 1 + \frac{p_2}{100} \cdot 2 + \frac{p_3}{100} \cdot 3 + \ldots = \Sigma \frac{p_i}{100} \cdot i$$

Dabei stellen $p_1$, $p_2$, $p_3$ ... bzw. $p_i$ den Anteil der einfach, zweifach dreifach ... i-fach glucosylierten Produkte in Gewichtsprozenten dar. Erfindungsgemäß vorteilhaft werden Produkte mit Glucosylierungsgraden von 1-2, insbesondere vorteilhaft von 1, 1 bis 1,5, ganz besonders vorteilhaft von 1,2-1,4, insbesondere von 1,3 gewählt.

Der Wert DP trägt den Umstande Rechnung, dass Alkylglucoside herstellungsbedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40-70 Gew.-%.

Erfindungsgemäß besonders vorteilhaft verwendete Alkylglylcoside werden gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren® 1200 (Henkel KGaA), Oramix®) NS 10 (Seppic).

Die Acyllactylate werden ihrerseits vorteilhaft gewählt aus der Gruppe der Substanzen, welche sich durch die Strukturformel

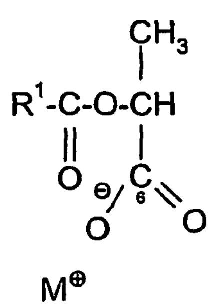

auszeichnen, wobei $R^1$ einen verzweigten oder unverzweigten Alkylrest mit 1 bis 30 Kohlenstoffatomen bedeutet und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumisostearyllactylat, beispielsweise das Produkt Pathionic® ISL von der Gesellschaft American Ingredients Company.

Die Betaine werden vorteilhaft gewählt aus der Gruppe der Substanzen, welche sich durch die Strukturformel

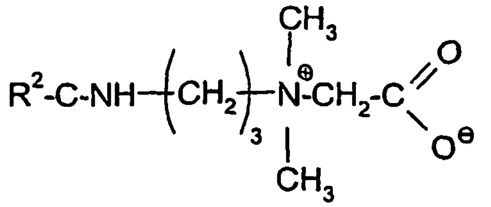

auszeichnen, wobei $R^2$ einen verzweigten oder unverzeigten Alkylrest mit 1 bis 30 Kohlenstoffatomen bedeutet.

Insbesondere vorteilhaft bedeutet $R^2$ einen verzweigten oder unverzweigten Alkylrest mit 6 bis 12 Kohlenstoffatomen.

Vorteilhaft ist beispielsweise Capramidopropylbetain, beispielsweise das Produkt Tego® Betain 810 von der Gesellschaft Th. Goldschmidt AG.

Als erfindungsgemäß vorteilhaftes Cocoamphoacetat wird beispielsweise Natriumcocoamphoacetat gewählt, wie es unter der Bezeichnung Miranol® Ultra C32 von der Gesellschaft Miranol Chemical Corp. erhältlich ist.

Die erfindungsgemäßen Zubereitungen sind vorteilhaft **dadurch gekennzeichnet, dass** das oder die hydrophilen Tenside in Konzentrationen von 0,01-20 Gew.-% bevorzugt 0,05-10 Gew.-%, besonders bevorzugt 0,1-5 Gew.-%, jeweils bezogen auf das Gesamtgewicht der Zusammensetzung, vorliegt oder vorliegen.

Zu Anwendung werden die erfindungsgemäßen kosmetischen und dermatologischen Zubereitungen in der für Kosmetika üblichen Weise auf die Haut und/oder die Haare in ausreichender Menge aufgebracht.

Als Emulgatoren können beispielsweise die bekannten W/O- und OIW-Emulgatoren verwendet werden. Es ist vorteilhaft, weitere übliche Co-emulgatoren in den erfindungsgemäßen bevorzugten O/W-Emulsionen zu verwenden.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylenglycol(13)stearytether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17),Polyethylenglycol(18) stearylether (Steareth-18), Polyethylenglycol(19)stearylether (Steareth-19), Polyethylenglycol(20)-stearylether (Steareth-20), Polyethylenglycol(12)isostearylether (Isosteareth-12), Polyethylenglycol(13)isostearylether (Isosteareth-13), Polyethylenglycol(14)-isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20) isostearylether (Isosteareth-20), Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylen-glycol(20)cetylether (Ceteth-20), Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20), Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15), Polyethylenglycol(12)laurylether (Laureth-12), Polyethylenglycol(12)-isolaurylether (Isolaureth-12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)-cetylstearylether (Ceteareth-19), Polyethylenglycol(20)cetylstearylether (Ceteareth-20).

Es ist ferner von Vorteil, die Fettsäureethoxylate aus folgender Gruppe zu wählen: Polyethylenglycol(20)stearat, Polyethylenglycol(21)stearat,

Polyethylenglycol(22)stearat, Polyethylenglycol(23)stearat, Polyethylenglycol(24)stearat, Polyethylenglycol(25)stearat, Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat, Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat,

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25) Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20) glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:

> Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C--Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkhole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Erfindungsgemäß bevorzugte Zubereitungen eignen sich besonders zum Schutz menschlicher Haut gegen UV-induzierte Alterungsprozesse sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer oder ölig-wässriger Gele bzw. Lösungen, als feste Stifte vorliegen oder als Aerosol konfektioniert sein.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen

Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und außer der oder den Verbindungen der Formel I beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die erfindungsgemäße Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen fotochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann verschiedene, in diesem Mitteltyp verwendete Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Im folgenden wird die Erfindung anhand von Beispielen näher erläutert.

## Beispiele

### Beispiel 1a Herstellung von nano-$TiO_2$

710 mL Natriumtitanat (Gehalt 140 g $TiO_2$/L ), erhalten durch Umsetzung von Metatitansäure mit Natronlauge, wird mit 100mL Wasser verdünnt und durch Zugabe von Salzsäure unter Bildung von Titandioxid (Rutil) bei pH 2,2 - 2,6 zersetzt. Dieses durch die Zersetzung erhaltene nanopartikuläre Titandioxid wird unter Zugabe von 115 mL 30 %iger Salzsäure peptisiert und durch weitere Wasserzugabe auf 1000 mL-Gesamtvolumen ergänzt. Die Peptisation erfolgt in einem geschlossenem Glaskolben bei 105°C über einem Zeitraum von 2 h. Das Produkt zeigt nadelförmige Kristallite.

### Beispiel 1b Herstellung von nano-$TiO_2$

Das aus Versuch 1a erhaltene Versuchsprodukt wird nach beendeter Peptisation für die Dauer von 2 h einer weiteren Hydrothermalbehandlung in einem Druckbehälter bei einer Temperatur von 180°C unterzogen. Das resultierende Produkt zeigt ovale Kristallite.

### Beispiel 2a: Beschichtung des nano-$TiO_2$ mit $SiO_2$

1 L der wässrigen, salzsauren Suspension des $TiO_2$ aus Beispiel 1 b wird mit NaOH auf einen pH-Wert von 6,5 gebracht und auf 80°C erhitzt. Anschließend werden zu der Suspension 52 mL Wasserglaslösung (entsprechend 384 g $SiO_2$ / L) bei konstantem pH-Wert (pH = 6,5 $\pm$ 0,5 ; Regelung durch Zugabe von $H_2SO_4$) zugegeben. Nach erfolgter Zugabe wird bei pH = 6,8 und 80°C 2 Stunden gerührt. Das Produkt wird anschließend auf eine Leitfähigkeit kleiner 100 $\mu$S/cm gewaschen und getrocknet.

### Beispiel 2b: Beschichtung des nano-$TiO_2$ mit $SiO_2$

1 L der wässrigen, salzsauren Suspension des $TiO_2$ aus Beispiel 1 b wird mit NaOH auf einen pH-Wert von 9,0 gebracht und auf 80°C erhitzt. Anschließend werden zu der Suspension 52 mL Wasserglaslösung (entsprechend

384 g $SiO_2$ / L) bei konstantem pH-Wert (pH = 9,0 ± 0,5, Regelung durch Zugabe von $H_2SO_4$) zugegeben. Nach erfolgter Zugabe wird bei pH = 6,8 und 80°C 2 Stunden gerührt. Das Produkt wird anschließend auf eine Leitfähigkeit kleiner 100 µS/cm gewaschen und getrocknet.

**Beispiel 2c: Beschichtung des nano-$TiO_2$ mit $SiO_2$**

1 L der wässrigen, salzsauren Suspension des $TiO_2$ aus Beispiel 1b wird mit NaOH auf einen pH-Wert von 2,0 gebracht und auf 80°C erhitzt. Anschließend werden zu der Suspension 52 mL Wasserglaslösung (entsprechend 384 g $SiO_2$ / L) bei konstantem pH-Wert (pH = 2,0 ± 0,5, Regelung durch Zugabe von $H_2SO_4$) zugegeben. Nach erfolgter Zugabe wird bei pH = 6,8 und 80°C 2 Stunden gerührt. Das Produkt wird anschließend auf eine Leitfähigkeit kleiner 100 µS/cm gewaschen und getrocknet.

**Beispiel 2d: Beschichtung des nano-$TiO_2$ mit $SiO_2$**

1 L der wässrigen, salzsauren Suspension des $TiO_2$ aus Beispiel 1b wird mit NaOH auf einen pH-Wert von 9,0 gebracht und auf 80°C erhitzt. Anschließend werden zu der Suspension 52 mL Wasserglaslösung (entsprechend 384 g $SiO_2$ / L) zugegeben. Der pH- Wert steigt dabei auf ca. 10,6 an. Nach erfolgter Zugabe wird durch Zugabe von Schwefelsäure auf pH 6,5 abgesenkt und danach bei pH = 6,8 und 80°C 2 Stunden gerührt. Das Produkt wird anschließend auf eine Leitfähigkeit kleiner 100 µS/cm gewaschen und getrocknet.

**Beispiel 3: Herstellung einer öligen Titandioxid Dispersion**

Zur Herstellung erfindungsgemäßer Titandioxid-Dispersionen werden die Produkte aus Beispiel 2 bzw. Eusolex®T-AVO (Handelsprodukt der Merck KGaA, Darmstadt) eingesetzt.

Durch Mischen der Bestandteile wird folgende Dispersion erzeugt:

37 Gew.% $SiO_2$-beschichtetes Titandioxid
5 Gew.-% Polyglyceryl-2 Dipolyhydroxystearate (Dehymuls® PGPH, Fa. Cognis)
58 Gew.-% Butylene Glycol Dicaprylate/Dicaprate (Miglyol® 8810; Fa. Sasol)

Dabei werden das Polyglyceryl-2 Dipolyhydroxystearate und das Butylene Glycol Dicaprylate/Dicaprate vorgemischt und anschließend mit dem $SiO_2$-beschichteten Titandioxid vermischt.

Die Dispersion auf Basis von Eusolex® T-AVO wird im folgenden auch als Eusolex® T-Oleo bezeichnet.

Eusolex® T-Oleo ist nach 1 Monat Lagerung bei 40°C so fest, dass es beim Stürzen nicht aus einem Becherglas fließt. Bei Scherung verflüssigt sich das Produkt wieder.

**Beispiel 4: Herstellung einer öligen Titandioxid Dispersion die zusätzlich organische UV Filter enthält**

Die Dispersion aus Beispiel 3 wird mit einem oder mehreren der organischen UV Filter der nachfolgenden Liste (es werden die INCI-Bezeichnungen angegeben) gemischt:

Butylmethoxy Dibenzoylmethane, Benzophenone-3, 4-Methylbenzylidene Camphor, Bis-Ethylhexyloxyphenol Methoxyphenyl Triazine, Ethylhexyl Triazone, Diethylamino Hydroxybenzoyl Hexyl Benzoate.

Dabei werden unter anderem folgende Zubereitungen erhalten:

Beispiel 4a:

| | |
|---|---|
| Benzophenone-3 | 11 Gew.-% |
| Dispersion aus Beispiel 3 mit Eusotex® T-AVO | 89 Gew.-% |

Beispiel 4b:

| | |
|---|---|
| 4-Methylbenzylidene Camphor | 15 Gew.-% |
| Dispersion aus Beispiel 3 mit Eusolex® T-AVO | 85 Gew.-% |

Beispiel 4c:

| | |
|---|---|
| Butylmethoxydibenzoylmethane | 7 Gew.-% |
| Dispersion aus Beispiel 3 mit Eusolex® T-AVO | 93 Gew.-% |

Beispiel 4d:

| | |
|---|---|
| Bis-Ethylhexyloxyphenol Methoxyphenyl Triazine | 2 Gew.-% |
| Dispersion aus Beispiel 3 mit Eusolex® T-AVO | 98 Gew.-% |

Beispiel 4e:

| | |
|---|---|
| Ethylhexyl Triazone | 2 Gew.-% |
| Dispersion aus Beispiel 3 mit Eusolex® T-AVO | 98 Gew.-% |

Beispiel 4f:

| | |
|---|---|
| Diethylamino Hydroxybenzoyl Hexyl Benzoate | 2 Gew.-% |
| Dispersion aus Beispiel 3 mit Eusolex® T-AVO | 98 Gew.-% |

Beispiel 4g:

| | |
|---|---|
| Uvinul® A Plus (Fa. BASF) | 7 Gew.-% |
| Dispersion aus Beispiel 3 mit Eusolex® T-AVO | 93 Gew.-% |

**Beispiel 5: Formulierungsbeispiele**

Im folgenden werden kosmetische Zubereitungen angegeben, die erfindungsgemäße ölige Titandioxid-dispersionen (jeweils als Eusolex® T-Oleo bezeichnet) enthalten. Im übrigen sind die INCI-Bezeichnungen der handelsüblichen Verbindungen angegeben.

**Sonnenschutzlotion (W/O)**

| **Inhaltsstoffe** | **INCI** | **[Gew.-%]** |
|---|---|---|
| A | | |
| Eusolex® T-Oleo | BUTYLENE GLYCOL DICAPRYLATE/DICAPRATE, TITANIUM DIOXIDE, SILICA, POLYGLYCERYL-2 DI POLYHYDROXYSTEARATE | 27.00 |
| Eusolex® OCR | OCTOCRYLENE | 5.50 |

(fortgesetzt)

| Inhaltsstoffe | INCI | [Gew.-%] |
|---|---|---|
| Eusolex® 9020 | BUTYL METHOXYDIBENZOYLMETHANE | 3.00 |
| Arlacel P135 | PEG-30 DIPOLYHYDROXYSTEARATE | 2.50 |
| Abil WE 09 | POLYGLYCERYL-4 ISOSTEARATE, CETYL PEG/PPG-10/1 DIMETHICONE, HEXYL LAURATE | 2.50 |
| Crodafos CES | CETEARYL ALCOHOL, DICETYL PHOSPHATE, CETETH-10 PHOSPHATE | 1.00 |
| Dow Corning 345 | CYCLOMETHICONE | 0.50 |
| Dow Corning 200 (100cs) | DIMETHICONE | 0.50 |
| Shea butter | BUTYROSPERMUM PARKII (SHEA BUTTER) | 1.00 |
| Paracera M | MICROWAX | 0.50 |
| B | | |
| Eusolex® UV-Pearls™ OMC | AQUA (WATER), ETHYLHEXYL METHOXYCINNAMATE, SILICA, PVP, CHLORPHENESIN, BHT | 20.00 |
| RonaCare® Ectoin | ECTOIN | 0.30 |
| RonaCare® Allantoin | ALLANTOIN | 0.20 |
| 1,2-Propanediol | PROPYLENE GLYCOL | 3.00 |
| Sodium chloride | SODIUM CHLORIDE | 0.40 |
| Titriplex® III | DISODIUM EDTA | 0.05 |
| Water, demineralized | AQUA (WATER) | 31.35 |
| C | | |
| Phenonip | PHENOXYETHANOL, BUTYLPARABEN, ETHYLPARABEN, PROPYLPARABEN, METHYLPARABEN | 0.70 |
| Fragrance (q.s.) | PARFUM | 0.00 |

**Sonnenschutzlotion (O/W)**

| | Inhaltsstoffe | INCI | [Gew.-%] |
|---|---|---|---|
| A | Eusolex® T-Oleo | BUTYLENE GLYCOL DICAPRYLATE/DICAPRATE, TITANIUM DIOXIDE, SILICA, POLYGLYCERYL-2 DIPOLYHYDROXYSTEARATE | 13.00 |
| | Emulsifier E 2155 | STEARETH-10, STEARETH-7, STEARYL ALCOHOL | 3.00 |
| | Teginacid H | GLYCERYL STEARATE, CETETH-20 | 3.00 |
| | Imwitor 900 | GLYCERYL STEARATE | 3.00 |
| | Paracera M | MICROWAX | 1.00 |
| | Cetiol | OLEYL OLEATE | 4.00 |
| | Luvitol EHO | CETEARYL OCTANOATE | 8.00 |
| | Miglyol 812 N | CAPRYLIC/CAPRIC TRIGLYCERIDE | 4.00 |
| | Propyl-4-hydroxybenzoate | PROPYLPARABEN | 0.05 |
| B | 1,2-Propanediol | PROPYLENE GLYCOL | 4.00 |
| | RonaCare™ Allantoin | ALLANTOIN | 0.20 |
| | Methyl-4-hydroxybenzoate | METHYLPARABEN | 0.15 |
| | Water, demineralized | AQUA (WATER) | 56,60 |

**Leichte Sonnenschutzcreme (O/W)**

| Inhaltsstoffe | INCI | [Gew.-%] |
|---|---|---|
| A | | |
| Eusolex® T-OLEO | BUTYLENE GLYCOL DICAPRYLATE/DICAPRATE, TITANIUM DIOXIDE, SILICA, POLYGLYCERYL-2 DIPOLYHYDROXYSTEARATE | **13,50** |
| Arlacel 165 | PEG-100 STEARATE, GLYCERYL STEARATE | 10,00 |
| Paraffin liquid | PARAFINUM LIQUIOUM (MINERAL OIL) | 16,50 |
| Lanette 16 | CETYL ALCOHOL | 2,00 |
| Lanolin | LANOLIN ANHYDROUS | 2,00 |
| Oxynex 2004 | BHT (and) GLYCERYL STEARATE (and) GLYCERYL OLEATE (and) ASCORBYL PALMITAT (and) CITRIC ACID (and) PROPYLENE GLYCOL | 0,05 |
| Propyl-4-hydroxybenzoat | PROPYLPARABENE | 0,05 |
| B | | |
| Sorbitol F liquid | SORBITOL | 3,00 |
| Glycerol (about 87%) | GLYCERIN | 2,00 |
| Titriplex III | DISODIUM EDTA | 0,05 |
| Methyl-4-hydroxybenzoat | METHYLPARABENE | 0,15 |
| Water, demineralized | AQUA (WATER) | 50,70 |

Für diese leichte Sonnenschutzcreme ergibt sich ein "Boots UVA star rating" von 2 Sternen. (Bestimmt nach der in T. Rudolf "UV-A-Protection assessment of Sunscreens - a critical review on the UV-A- to UV-B-absorbance ratio", SÖFW-Journal 130 (9), 2004, S.28ff.)

**Sonnenschutzlotion (W/O)**

| Rohstoff | INCI | [Gew.-%] |
|---|---|---|
| A | | |
| Eusolex® T-Oleo | BUTYLENE GLYCOL DICAPRYLATE/DICAPRATE, TITANIUM DIOXIDE, SILICA, POLYGLYCERYL-2 DIPOLYHYDROXYSTEARATE | 12,50 |
| Eusolex® 2292 | ETHYLHEXYL METHOXYCINNAMATE, BHT | 7,00 |
| Eusolex® OCR | OCTOCRYLENE | 4,00 |
| Eusolex® 9020 | BUTYL METHOXYDIBENZOYLMETHANE | 3,50 |
| Arlacel P135 | PEG-30 DIPOLYHYDROXYSTEARATE | 2,50 |
| Magnesiumstearat | MAGNESIUM STEARATE | 0,50 |
| Vitamin E-Acetat (DL-alpha-Tocopherolacetat)/Ph Eu | TOCOPHERYL ACETATE | 1.00 |
| B | | |
| RonaCare® Ectoin | ECTOIN | 0,10 |
| RonaCare® Allantoin | ALLANTOIN | 0,10 |
| Magnesiumsulfat | MAGNESIUM SULFATE | 0,70 |
| Glycerin, wasserfrei | GLYCERIN | 2,00 |
| Titriplex® III | DISODIUM EDTA | 0,05 |
| Wasser, demineralisiert | AQUA (WATER) | 54,05 |
| C | | |

(fortgesetzt)

| Rohstoff | INCI | [Gew.-%] |
|---|---|---|
| Wacker Belsil CM 040 | CYCLOPENTASILOXANE | 10,00 |
| Aerosil R 972 | SILICA DIMETHYL SILYLATE | 1,00 |
| Phenonip | PHENOXYETHANOL, BUTYLPARABEN, ETHYLPARABEN, PROPYLPARABEN, METHYLPARABEN | 1,00 |
| Parfümöl (q.s.) | PARFUM | 0,00 |

**Sprühbare Sonnenmilch (W/O)**

| Rohstoff | INCI | [Gew.-%] |
|---|---|---|
| A | | |
| Eusolex® T-Oleo | BUTYLENE GLYCOL DICAPRYLATE/DICAPRATE, TITANIUM DIOXIDE, SILICA, POLYGLYCERYL-2 DI POLYHYDROXYSTEARATE | 7,50 |
| Eusolex® OCR | OCTOCRYLENE | 4,50 |
| Eusolex® 9020 | BUTYL METHOXYDIBENZOYLMETHANE | 2,00 |
| Arlacel P135 | PEG-30 DIPOLYHYDROXYSTEARATE | 3,00 |
| Arlamol HD | ISOHEXADECANE | 8,00 |
| Miglyol 812 N | CAPRYLICICAPRIC TRIGLYCERIDE | 4,00 |
| Arlamol DOA | DIOCTYL ADIPATE | 4,00 |
| Permethyl 104A | POLYISOBUTENE | 5,50 |
| B | | |
| RonaCare® Ectoin | ECTOIN | 0,10 |
| Magnesiumsulfat | MAGNESIUM SULFATE | 0,70 |
| Glycerin, wasserfrei | GLYCERIN | 4,00 |
| Wasser, demineralisiert | AQUA (WATER) | 55,70 |
| C | | |
| Germaben II | PROPYLENE GLYCOL, DIAZOLIDINYL UREA, METHYLPARABEN, PROPYLPARABEN | 1,00 |
| Parfümöl (q.s.) | PARFUM | 0,00 |

**Sonnenspray (O/W)**

| Rohstoff | INCI | [Gew.-%] |
|---|---|---|
| A | | |
| Eusolex® T-Oleo | BUTYLENE GLYCOL DICAPRYLATE/DICAPRATE, TITANIUM DIOXIDE, SILICA, POLYGLYCERYL-2 DIPOLYHYDROXYSTEARATE | 7,50 |
| Eusolex® OCR | OCTOCRYLENE | 5,00 |
| Eusolex® 9020 | BUTYL METHOXYDIBENZOYLMETHANE | 1,00 |
| Imwitor 377 | GLYCERYL COCOATE/ CITRATE/LACTATE | 4,00 |
| Imwitor 928 | GLYCERYL COCOATE | 3,00 |
| B | | |
| Wasser, demineralisiert | AQUA (WATER) | 77,80 |
| RonaCare® Ectoin | ECTOIN | 0,30 |
| C | | |

(fortgesetzt)

| Rohstoff | INCI | [Gew.-%] |
|---|---|---|
| Pemulen TR-2 | ACRYLATES/C10-30 ALKYL ACRYLATE CROSSPOLYMER | 0,10 |
| LaraCare A-200 | GALACTOARABINAN | 0,30 |
| | | |
| D | | |
| Parfümöl (q.s.) | PARFUM | 0,00 |
| Phenonip | PHENOXYETHANOL, BUTYLPARABEN, ETHYLPARABEN, PROPYLPARABEN, METHYLPARABEN | 1.00 |
| Natronlauge, 10 %ig | SODIUM HYDROXIDE | 0,00 |

**Sonnenmilch mit Perlglanzpigment (O/W)**

| Rohstoff | INCI | [Gew.-%] |
|---|---|---|
| A | | |
| Ronastar® Golden Sparks | CALCIUM ALUMINUM BOROSILICATE, SILICA, CI 77891 (TITANIUM DIOXIDE), TIN OXIDE | 2,00 |
| RonaCare® Ectoin | ECTOIN | 0,10 |
| Glycerin, wasserfrei | GLYCERIN | 3,00 |
| Wasser, demineralisiert | AQUA (WATER) | 65,15 |
| | | |
| B | | |
| Keltrol TF | XANTHAN GUM | 0,20 |
| Veegum Ultra | MAGNESIUM ALUMINUM SILICATE | 0,50 |
| | | |
| C | | |
| Eusolex® T-Oleo | BUTYLENE GLYCOL DICAPRYLATE/DICAPRATE, TITANIUM DIOXIDE, SILICA, POLYGLYCERYL-2 DI POLYHYDROXYSTEARATE | 8,00 |
| Eusolex® 2292 | ETHYLHEXYL METHOXYCINNAMATE, BHT | 7,00 |
| Eusolex® 4360 | BENZOPHENONE-3 | 3,00 |
| Arlacel 165 VP | GLYCERYL STEARATE, PEG-100 STEARATE | 3,00 |
| Lanette O | CETEARYL ALCOHOL | 2,00 |
| Cutina GMS | GLYCERYL STEARATE | 1,00 |
| Cutina CP | CETYL PALMITATE | 1,00 |
| Cetiol S | DIOCTYLCYCLOHEXANE | 3,00 |
| OL-a-Tocopherol | TOCOPHEROL | 0,05 |
| | | |
| D | | |
| Phenonip | PHENOXYETHANOL, BUTYLPARABEN, ETHYLPARABEN, PROPYLPARABEN, METHYLPARABEN | 1,00 |
| Parfümöl (q.s.) | PARFUM | 0,00 |

**Sprühbare Sonnenlotion (O/W**

| Rohstoff | INCI | [Gew.-%] |
|---|---|---|
| A | | |
| Ceralution H | BEHENYL ALCOHOL, GLYCERYL STEARATE, GLYCERYL STEARATE CITRATE, SODIUM | 2,00 |

(fortgesetzt)

| Rohstoff | INCI | [Gew.-%] |
|---|---|---|
| | DICOCOYLETHYLENEDIAMINE PEG-15 SULFATE | |
| Marlipal O 13/120 | TRIDECETH-12 | 2,50 |
| Lanol 99 | ISONONYL ISONONANOATE | 6,00 |
| Dow Corning 200 (100cs) | DIMETHICONE | 1,75 |
| Softisan 100 | HYDROGENATED COCO-GLYCERIDES | 1,50 |
| Sheabutter fest | BUTYROSPERMUM PARKII (SHEA BUTTER) | 1,00 |
| Vitamin E-Acetat (DL-alpha-Tocopherolacetat)/Ph Eu | TOCOPHERYL ACETATE | 0,10 |
| B | | |
| RonaCare® Ectoin | ECTOIN | 0,10 |
| Ceralution F | SODIUM LAUROYL LACTYLATE, SODIUM DICOCOYLETHYLENEDIAMINE PEG-15 SULFATE | 2,50 |
| Keltrol CGSFT (1%ig in Wasser) | AQUA, XANTHAN GUM | 10,00 |
| Glycerin, wasserfrei | GLYCERIN | 2,00 |
| Propylenglykol, 1,2- | PROPYLENE GLYCOL | 4,00 |
| Wasser, demineralisiert | AQUA (WATER) | 40,55 |
| Phenonip | PHENOXYETHANOL, BUTYLPARABEN, ETHYLPARABEN, PROPYLPARABEN, METHYLPARABEN | 1,00 |
| C | | |
| Eusolex® T-Oleo | BUTYLENE GLYCOL DICAPRYLATE/DICAPRATE, TITANIUM DIOXIDE, SILICA, POLYGLYCERYL-2 DIPOLYHYDROXYSTEARATE | 25,00 |
| Parfümöl (q.s.) | PARFUM | 0,00 |

**Sonnenschutzlotion (O/W)**

| Rohstoff | INCI | [Gew.-%] |
|---|---|---|
| A | | |
| Eusolex® T-Oleo | BUTYLENE GLYCOL DICAPRYLATE/DICAPRATE, TITANIUM DIOXIDE, SILICA, POLYGLYCERYL-2 DIPOLYHYDROXYSTEARATE | 3,00 |
| Eusolex® 9020 | BUTYL METHOXYDIBENZOYLMETHANE | 2,00 |
| Eusolex® OCR | OCTOCRYLENE | 2,00 |
| Stearinsäure | STEARIC ACID | 0,70 |
| Tegin M | GLYCERYL STEARATE | 0,70 |
| Tegosoft DC | DECYL COCOATE | 3,00 |
| Tegosoft CR | CETYL RICINOLEATE | 2,00 |
| RonaCare® Tocopherolacetat | TOCOPHERYL ACETATE | 0,50 |
| Antaron V-216 | PVP/HEXADECENE COPOLYMER | 0,50 |
| B | | |
| RonaCare® Ectoin | ECTOIN | 0,30 |
| Tego Care CG 90 | CETEARYL GLUCOSIDE | 1,50 |
| 1,2-Propandiol | PROPYLENE GLYCOL | 2,00 |
| Glycerin, wasserfrei | GLYCERIN | 1,00 |
| Wasser, demineralisiert | AQUA (WATER) | 79,30 |

(fortgesetzt)

| Rohstoff | INCI | [Gew.-%] |
|---|---|---|
| C | | |
| Tego Carbomer 141 | CARBOMER | 0,20 |
| Isopropylmyristat | ISOPROPYL MYRISTATE | 0,80 |
| D | | |
| Germaben II | PROPYLENE GLYCOL, DIAZOLIDINYL UREA, METHYLPARABEN, PROPYLPARABEN | 0,50 |
| Parfümöl (q.s.) | PARFUM | 0,00 |

**Sonnenschutzlotion (W/O)**

| Rohstoff | INCI | [Gew.-%] |
|---|---|---|
| A | | |
| Eusolex® T-Oleo | BUTYLENE GLYCOL DICAPRYLATE/DICAPRATE, TITANIUM DIOXIDE, SILICA, POLYGLYCERYL-2 DIPOLYHYDROXYSTEARATE | 6,50 |
| Abil EM 90 | CETYL PEG/PPG-10/1 DIMETHICONE | 2,50 |
| Abil Wax 9801 | CETYL DIMETHICONE | 1,00 |
| Gilugel SIL 5 | ALUMINIUMIMAGNESIUM HYDROXIDE STEARATE, CYCLOMETHICONE | 5,00 |
| Paracera W 80 | CERESIN (MICROCRYSTALLINE WAX) | 1,00 |
| Cutina HR | HYDROGENATED CASTOR OIL | 1,00 |
| Tegosoft DEC | DIETHYLHEXYL CARBONATE | 6,00 |
| Tegosoft TN | C12-15 ALKYL BENZOATE | 4,00 |
| Tegosoft OS | ETHYLHEXYL STEARATE | 2,50 |
| Dow Corning 345 | CYCLOMETHICONE | 10,00 |
| Prisorine 3505 | ISOSTEARIC ACID | 1,00 |
| RanaCare® Tocopherolacetat | TOCOPHERYL ACETATE | 0,50 |
| B | | |
| Eusolex® UV-Pearis™ OMC | AQUA (WATER), ETHYLHEXYL METHOXYCINNAMATE, SILICA, PVP, CHLORPHENESIN, BHT | 20,00 |
| RonaCare® Ectoin | ECTOIN | 0,20 |
| Glycerin, wasserfrei | GLYCERIN | 3,00 |
| Natriumchlorid | SODIUM CHLORIDE | 0,50 |
| Wasser, demineralisiert | AQUA (WATER) | 35,30 |
| Natronlauge, 10 %ig | SODIUM HYDROXIDE | 0,00 |
| C | | |
| Konservierungsmittel (q.s.) | | 0,00 |
| Parfümöl (q.s.) | PARFUM | 0,00 |

**Sonnenschutzmilch (W/O)**

| Rohstoff | INCI | [Gew.-%] |
|---|---|---|
| A | | |
| Eusolex® T-Oleo | BUTYLENE GLYCOL DICAPRYLATE/DICAPRATE, TITANIUM DIOXIDE, SILICA, POLYGLYCERYL-2 DIPOLYHYDROXYSTEARATE | 8,00 |
| Eusolex® 2292 | ETHYLHEXYL METHOXYCINNAMATE, BHT | 5,00 |

(fortgesetzt)

| Rohstoff | INCI | [Gew.-%] |
|---|---|---|
| Eusolex® OCR | OCTOCRYLENE | 5,00 |
| Eusolex® 4360 | BENZOPHENONE-3 | 5,00 |
| Abil EM 90 | CETYL PEG/PPG-10/1 DIMETHICONE | 2,50 |
| Cutina HR | HYDROGENATED CASTOR OIL | 0,50 |
| Prisorine 3505 | ISOSTEARIC ACID | 1,00 |
| Tegosoft OS | ETHYLHEXYL STEARATE | 11,00 |
| Isopropylmyristat | ISOPROPYL MYRISTATE | 6,00 |
| Arlamol HD | ISOHEXADECANE | 9,00 |
| Paracera W 80 | CERESIN (MICROCRYSTALLINE WAX) | 1,00 |
| Propyl-4-hydroxybenzoat | PROPYLPARABEN | 0,05 |
| B | | |
| RonaCare® Ectoin | ECTOIN | 0,30 |
| Natriumchlorid | SODIUM CHLORIDE | 0,50 |
| Wasser, demineralisiert | AQUA (WATER) | 45,15 |

**Shake before use - Sonnenschutzlotion (W/Si)**

| Rohstoff | INCI | [Gew.-%] |
|---|---|---|
| A | | |
| KSG-210 | DIMETHICONE, DIMETHICONE PEG-10/15 CROSSPOLYMER | 3,00 |
| KSG-15 | CYCLOPENTASILOXANE, DIMETHICONE/VINYL DIMETHICONE CROSSPOLYMER | 2,00 |
| DM-Fluid-A-6cs | DIMETHICONE | 5,00 |
| KF-995 | CYCLOPENTASILOXANE | 5,00 |
| KF-6028 | PEG-9 POLYDIMETHYLSILOXYETHYL DIMETHICONE | 1,00 |
| Crodamol TN | ISOTRIDECYL ISONONANOATE | 4,00 |
| B | | |
| Dow Corning 345 | CYCLOMETHICONE | 15,00 |
| Abil Wax 9801 | CETYL DIMETHICONE | 6,00 |
| Eusolex® T-Oleo | BUTYLENE GLYCOL DICAPRYLATE/DICAPRATE, TITANIUM DIOXIDE, SILICA, POLYGLYCERYL-2 DIPOLYHYDROXYSTEARATE | 39,00 |
| C | | |
| RonaCare® Ectoin | ECTOIN | 0,10 |
| 1,2-Propandiol | PROPYLENE GLYCOL | 2,00 |
| Natriumchlorid | SODIUM CHLORIDE | 1,00 |
| tri-Natriumcitrat-Dihydrat | SODIUM CITRATE | 0,20 |
| Wasser, demineralisiert | AQUA (WATER) | 16,70 |

**Feuchtigkeits Lotion mit UV-Schutz (O/W)**

| Rohstoff | INCI | [Gew.%] |
|---|---|---|
| A | | |
| Eusolex® T-Oleo | BUTYLENE GLYCOL DICAPRYLATE/DICAPRATE. TITANIUM DIOXIDE, SILICA, POLYGLYCERYL-2 DIPOLYHYDROXYSTEARATE | 1,00 |

(fortgesetzt)

| Rohstoff | INCI | [Gew.%] |
|---|---|---|
| Eusolex® 9020 | BUTYL METHOXYDIBENZOYLMETHANE | 5,00 |
| Eusolex® OCR | OCTOCRYLENE | 5,00 |
| Eusolex® OS | ETHYLHEXYL SALICYLATE | 5,00 |
| Tego Care 450 | POLYGLYCERYL-3 METHYLGLUCOSE DISTEARATE | 2,00 |
| Tegosoft TN | C12-15 ALKYL BENZOATE | 7,00 |
| Syncrowax HGLC | C18-36 ACID TRIGLYCERIDE | 1,75 |
| Antaron V-216 | PVPIHEXADECENE COPOLYMER | 1,75 |
| Sheabutter fest | BUTYROSPERMUM PARKII (SHEA BUTTER) | 0,50 |
| Dow Coming 200 (100cs) | DIMETHICONE | 2,00 |
| Tween 20 | POLYSORBATE 20 | 0,25 |
| Vitamin E-Acetat (DL-alpha-Tocopherolacetat)/Ph Eu | TOCOPHERYL ACETATE | 0,20 |
| Propyl-4-hydroxybenzoat | PROPYLPARABEN | 0,05 |
| B | | |
| RonaCare® Ectoin | ECTOIN | 0,30 |
| 1,3-Butandiol | BUTYLENE GLYCOL | 5,00 |
| Pemulen TR-2 CROSSPOLYMER | ACRYLATES/C10-30 ALKYL ACRYLATE | 0,15 |
| Glycerin, wasserfrei | GLYCERIN | 1,75 |
| Titriplex® III | DISODIUM EDTA | 0,05 |
| Wasser, demineralisiert | AQUA (WATER) | 61,10 |
| Methyl-4-hydroxybenzoat | METHYLPARABEN | 0,15 |
| Natronlauge, 10 %ig | SODIUM HYDROXIDE | 0,00 |
| Parfümöl (q.s.) | PARFUM | 0,00 |

**Anti-Aging Creme (O/W)**

| Rohstoff | NCI | [Gew.-%] |
|---|---|---|
| A | | |
| Eusolex® T-Oleo | BUTYLENE GLYCOL DICAPRYLATE/DICAPRATE, TITANIUM DIOXIDE, SILICA, POLYGLYCERYL-2 DIPOLYHYDROXYSTEARATE | 5,00 |
| Eusolex® OCR | OCTOCRYLENE | 2,00 |
| Eusolex® 9020 | BUTYL METHOXYDIBENZOYLMETHANE | 2,00 |
| Tego Care 215, Pellets | GLYCERYL STEARATE, CETEARETH-15 | 3,00 |
| Lanette 18 | STEARYL ALCOHOL | 2,50 |
| Luvitol EHO | CETEARYL OCTANOATE | 7,00 |
| Cetiol | OLEYL OLEATE | 7,00 |
| Paracera M | MICROWAX | 0,70 |
| Miglyol 812 N | CAPRYLIC/CAPRIC TRIGLYCERIDE | 7,80 |
| Abil 350 | DIMETHICONE | 0,50 |
| RonaCare® Tocopherolacetat | TOCOPHERYL ACETATE | 0,50 |
| Propyl-4-hydroxybenzoat | PROPYLPARABEN | 0,05 |
| B | | |
| RonaCare® Ectoin | ECTOIN | 0,30 |
| 1,2-Propandiol | PROPYLENE GLYCOL | 4,00 |
| Methyl-4-hydroxybenzoat | METHYLPARABEN | 0,15 |

(fortgesetzt)

| Rohstoff | NCI | [Gew.-%] |
|---|---|---|
| Wasser, demineralisiert | AQUA (WATER) | 57,30 |
| C | | |
| RonaCare® VTA | AQUA (WATER), ALCOHOL DENAT., LECITHIN. DISODIUM RUTINYL DISULFATE, HYDROXYPROLINE SORBITOL, MAGNESIUM ASCORBYL PHOSPHATE, TOCOPHEROL, ASCORBYL PALMITATE, GLYCERYL STEARATE, GLYCERYL OLEATE, CITRIC ACID | 2,00 |
| Parfümöl Xanadu | PARFUM | 0,20 |

**Sonnenschutzlotion O/W**

| Rohstoff | INCI | [Gew.-%] |
|---|---|---|
| A | | |
| Eusolex® T-Oleo | BUTYLENE GLYCOL DICAPRYLATE/DICAPRATE, TITANIUM DIOXIDE, SILICA, POLYGLYCERYL-2 DIPOLYHYDROXYSTEARATE | 13,50 |
| Imwitor 372 P | GLYCERYL STEARATE CITRATE | 2,50 |
| Lanette 18 | STEARYL ALCOHOL | 1,50 |
| Softisan 100 | HYDROGENATED COCO-GLYCERIDES | 1,00 |
| Cetiol OE | DICAPRYLYL ETHER | 3,00 |
| Eutanol G | OCTYLDODECANOL | 3,00 |
| Dow Corning 345 | CYCLOMETHICONE | 2,50 |
| Antaron V-216 | PVP/HEXADECENE COPOLYMER | 0,50 |
| Vitamin E-Acetat (DL-alpha-Tocopherolacetat)/Ph Eu | TOCOPHERYL ACETATE | 0,50 |
| Propyl-4-hydroxybenzoat | PROPYLPARABEN | 0,05 |
| B | | |
| Keltrol RD | XANTHAN GUM | 0,15 |
| RonaCare® Ectoin | ECTOIN | 0,10 |
| Glycerin, wasserfrei | GLYCERIN | 5,00 |
| Titriplex® III | DISODIUM EDTA | 0,05 |
| Wasser, demineralisiert | AQUA (WATER) | 65,70 |
| Methyl-4-hydroxybenzoat | METHYLPARABEN | 0,15 |
| C | | |
| Cetiol OE | DICAPRYLYL ETHER | 0,50 |
| Pemulen TR-2 CROSSPOLYMER | ACRYLATES/C10-30 ALKYL ACRYLATE | 0,10 |
| Triethanolamin | TRIETHANOLAMINE | 0,20 |
| Parfümöl (q.s.) | PARFUM | 0,00 |

**Sonnenschutz Lotion - sehr hoher Lichtschutzfaktor (O/W)**

| Rohstoff | INCI | [Gew.-%] |
|---|---|---|
| A | | |
| Eusolex® T-Oleo | BUTYLENE GLYCOL DICAPRYLATE/DICAPRATE, TITANIUM DIOXIDE, SILICA, POLYGLYCERYL-2 | 13,00 |

(fortgesetzt)

| Rohstoff | INCI | [Gew.-%] |
|---|---|---|
| | DIPOLYHYDROXYSTEARATE | |
| Eusolex® OCR | OCTOCRYLENE | 10,00 |
| Eusolex®) 9020 | BUTYL METHOXYDIBENZOYLMETHANE | 4,00 |
| Eusolex® 4360 | BENZOPHENONE-3 | 4,00 |
| Emulgade F | SODIUM CETEARYL SULFATE, CETEARYL ALCOHOL, PEG-40 CASTOR OIL | 3,00 |
| Tegin | GLYCERYL STEARATE SE | 1,50 |
| Syncrowax HGLC | C18-36 ACID TRIGLYCERIDE | 1,50 |
| Softisan 100 | HYDROGENATED COCO-GLYCERIDES | 1,50 |
| Dow Coming 345 | CYCLOMETHICONE | 6,00 |
| X-Tend 226 | PHENYLETHYL BENZOATE | 4,50 |
| Antaron V-216 | PVPIHEXADECENE COPOLYMER | 1,00 |
| Vitamin E-Acetat (DL-alpha-Tocopherolacetat)/Ph Eu | TOCOPHERYL ACETATE | 0,50 |
| Propyl-4-hydroxybenzoat | PROPYLPARABEN | 0,05 |
| B | | |
| Eusolex® 232 | PHENYLBENZIMIDAZOLE SULFONIC ACID | 3,00 |
| Triethanolamin | TRIETHANOLAMINE | 1,60 |
| Keltrol RD | XANTHAN GUM | 0,20 |
| RonaCare® Ectoin | ECTOIN | 0,10 |
| Glycerin, wasserfrei | GLYCERIN | 5,00 |
| Titriplex® III | DISODIUM EDTA | 0,05 |
| Methyl-4-hydroxybenzoat | METHYLPARABEN | 0,15 |
| Wasser, demineralisiert | AQUA (WATER) | 39,35 |
| Parfümöl (q.s.) | PARFUM | 0,00 |

**Sonnenschutzspray (O/W)**

| Rohstoff | INCI | [Gew.-%] |
|---|---|---|
| A | | |
| Keltrol TF | XANTHAN GUM | 0,10 |
| Veegum Ultra | MAGNESIUM ALUMINUM SILICATE | 0,25 |
| RonaCate® Ectoin | ECTOIN | 0,10 |
| Glycerin, wasserfrei | GLYCERIN | 5,00 |
| Natronlauge, 10 %ig | SODIUM HYDROXIDE | 0,85 |
| Methyl-4-hydroxybenzoat | METHYLPARABEN | 0,15 |
| Wasser, demineralisiert | AQUA (WATER) | 34,85 |
| A1 | | |
| Dermacryl AQF | ACRYLATES COPOLYMER | 4,40 |
| B | | |
| Eusolex® T-Oleo | BUTYLENE GLYCOL DICAPRYLATE/DICAPRATE, TITANIUM DIOXIDE, SILICA, POLYGLYCERYL-2 DIPOLYHYDROXYSTEARATE | 3,50 |
| Eusolex® 2292 | ETHYLHEXYL METHOXYCINNAMATE, BHT | 7,50 |
| Eusolex® 4360 | BENZOPHENONE-3 | 2,50 |
| Emulgade SE | ACETYL PALMITATE, GLYCERYL STEARATE, CETEARETH-20, CETEARETH-12, CETEARYL | 4,60 |

(fortgesetzt)

| Rohstoff | INCI | [Gew.-%] |
|---|---|---|
| | ALCOHOL | |
| Eumulgin B 2 | CETEARETH-20 | 3,90 |
| Estol 3609 | TRIETHYLHEXANOIN | 2,00 |
| Propyl-4-hydroxybenzoat | PROPYLPARABEN | 0,05 |
| Parfümöl Xanadu | PARFUM | 0,25 |
| C | | |
| Wasser, demineralisiert | AQUA (WATER) | 30,00 |

**Sonnenmilch mit Insektenschutz (O/W)**

| Rohstoff | INCI | [Gew.-%] |
|---|---|---|
| IR3535® | ETHYL BUTYLACETYLAMINOPROPIONATE | 5,00 |
| Eusolex® T-Oleo | BUTYLENE GLYCOL DICAPRYLATE/DICAPRATE, TITANIUM DIOXIDE, SILICA, POLYGLYCERYL-2 DIPOLYHYDROXYSTEARATE | 2,50 |
| Eusolex® 2292 | ETHYLHEXYL METHOXYCINNAMATE, BHT | 7,50 |
| Eusolex® OCR | OCTOCRYLENE | 10,00 |
| Eusolex® 4360 | BENZOPHENONE-3 | 2,00 |
| RonaCare® Tocopherolacetat | TOCOPHERYL ACETATE | 0,25 |
| Arlacel 165 VP | GLYCERYL STEARATE, PEG-100 STEARATE | 3,20 |
| Montanov S | COCO-GLUCOSIDE, COCONUT ALCOHOL | 1,30 |
| Cetiol B | DIBUTYL ADIPATE | 5,50 |
| B | | |
| Sepigel 305 | LAURETH-7, POLYACRYLAMIDE, C13-14 ISOPARAFFIN | 1,20 |
| C | | |
| Glycerin (87 % reinst) | GLYCERIN | 7,00 |
| RonaCare® Ectoin | ECTOIN | 0,20 |
| Keltrol CG-SFT | XANTHAN GUM | 0,15 |
| Veegum | MAGNESIUM ALUMINUM SILICATE | 0,20 |
| Titriplex® III | DISODIUM EDTA | 0,10 |
| Wasser, demineralisiert | AQUA (WATER) | 52,80 |
| D | | |
| Germaben II | PROPYLENE GLYCOL, DIAZOLIDINYL UREA, METHYLPARABEN,PROPYLPARABEN | 1,00 |
| Parfümöl (q.s.) | PARFUM | 0,00 |
| E | | |
| Natronlauge, 10 %ig | SODIUM HYDROXIDE | 0,10 |

**Sonnenschutzlotion sensitiv W/O**

| Rohstoff | INCI | [Gew.-%] |
|---|---|---|
| A | | |
| Eusolex® T-Oleo | BUTYLENE GLYCOL DICAPRYLATE/DICAPRATE, TITANIUM DIOXIDE, SILICA, POLYGLYCERYL-2 | 12,00 |

(fortgesetzt)

| Rohstoff | INCI | [Gew.-%] |
|---|---|---|
| | DIPOLYHYDROXYSTEARATE | |
| Eusolex® OCR | OCTOCRYLENE | 5.00 |
| Eusolex® 9020 | BUTYL METHOXYDIBENZOYLMETHANE | 2,00 |
| Tegosoft TN | C12-15 ALKYL BENZOATE | 5,00 |
| Tego-Soft Cl | CETEARYL ISONONANOATE | 4,00 |
| Dehymuls PGPH | POLYGLYCERYL-2 DIPOLYHYDROXYSTEARATE | 3,00 |
| Bienenwachs gebleicht | CERA ALBA (BEESWAX) | 2,00 |
| Zinkstearat | ZINC STEARATE | 1,00 |
| Monomuls 90-O 18 | 1,00 | |
| Softisan 100 | HYDROGENATED COCO-GLYCERIDES | 0,50 |
| Vitamin E-Acetat (DL-alpha-Tocopherolacetat)/Ph Eu | TOCOPHERYL ACETATE | 1,00 |
| B | | |
| RonaCare® Ectoin | ECTOIN | 0,20 |
| RonaCare® Bisabolol | BISABOLOL | 0,50 |
| Glycerin, wasserfrei | GLYCERIN | 4,00 |
| Magnesiumsulfat | MAGNESIUM SULFATE | 1,00 |
| Wasser, demineralisiert | AQUA (WATER) | 56,30 |
| RanaCare® Bisabolol | BISABOLOL | 0,50 |
| Parfümöl (q.s.) | PARFUM | 0,00 |
| Germaben II | PROPYLENE GLYCOL, DIAZOLIDINYL UREA, METHYLPARABEN,PROPYLPARABEN | 1,00 |

**Sonnenschutzlotion (O/W)**

| Rohstoff | INCI | [Gew.-%] |
|---|---|---|
| A | | |
| Eusolex® T-Oleo | BUTYLENE GLYCOL DICAPRYLATE/DICAPRATE, TITANIUM DIOXIDE, SILICA, POLYGLYCERYL-2 DIPOLYHYDROXYSTEARATE | 4,00 |
| Eusolex® OCR | OCTOCRYLENE | 6,00 |
| Eusolex® 9020 | BUTYL METHOXYDIBENZOYLMETHANE | 2,00 |
| Arlacel 165 VP | GLYCERYL STEARATE, PEG-100 STEARATE | 2,00 |
| Amphisol K | POTASSIUM CETYL PHOSPHATE | 1,50 |
| Lanette 16 | CETYL ALCOHOL | 1,00 |
| Stearinsäure | STEARIC ACID | 1,00 |
| Sheabutter fest | BUTYROSPERMUM PARKII (SHEA BUTTER) | 0,50 |
| Dow Corning 245 | CYCLOMETHICONE | 4,00 |
| Arlamol HD | ISOHEXADECANE | 2,00 |
| Dow Corning 200 (50cs) | DIMETHICONE | 0,50 |
| Antaron V-216 | PVP/HEXADECENE COPOLYMER | 1,00 |
| Vitamin E-Acetat (DL-alpha-Tocopherolacetat)/Ph Eu | TOCOPHERYL ACETATE | 1,00 |
| B | | |
| Eusolex®) UV-Pearls™ OMC | AQUA (WATER), ETHYLHEXYL METHOXYCINNAMATE, SILICA, PVP, CHLORPHENESIN, BHT | 3,00 |
| RonaCare® Ectoin | ECTOIN | 0,15 |
| Glycerin, wasserfrei | GLYCERIN | 5,00 |

(fortgesetzt)

| Rohstoff | INCI | [Gew.-%] |
|---|---|---|
| Propylenglykol, 1,2- | PROPYLENE GLYCOL | 4,00 |
| Keltrol CG-SFT | XANTHAN GUM | 0,10 |
| Wasser, demineralisiert | AQUA (WATER) | 59,70 |
| | | |
| C | | |
| Pemulen TR-2 | ACRYLATES/C10-30 ALKYL ACRYLATE CROSSPOLYMER | 0,05 |
| Celiol LC | COCO-CAPRYLATE/CAPRATE | 0,50 |
| | | |
| Triethanolamin | TRIETHANOLAMINE | 0,00 |
| Liquapar PE | PHENOXYETHANOL, ISOPROPYLPARABEN, ISOBUTYLPARABEN. BUTYLPARABEN | 1,00 |
| Parfümöl (q.s.) | PARFUM | 0,00 |

**Beispiel 6: Rezepturbeispiele**

Im folgenden werden beispielhaft Rezepturen für kosmetische Zubereitungen angegeben, die in gleicher Weise mit Eusolex® T-Oleo erhalten werden können. (In der Tabelle jeweils als T-Oleo bezeichnet). Im übrigen sind die INCI-Bezeichnungen der handelsüblichen Verbindungen angegeben.

UV-Pearl, OMC steht für die Zubereitung mit der INCI-Bezeichnung:

Water (for EU: Aqua), Ethylhexyl Methoxycinnamate, Silica, PVP, Chlorphenesin, BHT; diese Zubereitung ist im handel unter der Bezeichnung Eusolex®UV Pearl™OMC von der Merck KGaA, Darmstadt erhältlich.
Die anderen in den Tabellen angegebenen UV-Pearl sind jeweils analog zusammengesetzt, wobei OMC gegen die angegebenen UV-Filter ausgetauscht wurde.

Tabelle 1 W/O-Emulsionen (Zahlen in Gew.-%)

| | 1-1 | 1-2 | 1-3 | 1-4 | 1-5 | 1-6 | 1-7 | 1-8 | 1-9 | 1-10 |
|---|---|---|---|---|---|---|---|---|---|---|
| T-Oleo | 3 | 2 | 5 | 10 | 7 | 4 | 15 | 1 | 3 | 3 |
| Butylmethoxydibenzoyl-methane | 5 | 3 | 2 | 1 | 2 | | | | 1 | 1 |
| Zinc oxide | | | | | | | | 5 | 2 | |
| UV-Pearl , OMC | 30 | 15 | 15 | 15 | 15 | 15 | 15 | 15 | 15 | 15 |
| Polyglyceryl-3-Dimerate | 3 | 3 | 3 | 3 | 3 | 3 | 3 | 3 | 3 | 3 |
| Cera Alba | 0,3 | 0,3 | 0,3 | 0,3 | 0,3 | 0,3 | 0,3 | 0,3 | 0,3 | 0,3 |
| Hydrogenated Castor Oil | 0,2 | 0,2 | 0,2 | 0,2 | 0,2 | 0,2 | 0,2 | 0,2 | 0,2 | 0,2 |
| Paraffinium Liquidum | 7 | 7 | 7 | 7 | 7 | 7 | 7 | 7 | 7 | 7 |
| Caprylic/Capric Triglyceride | 7 | 7 | 7 | 7 | 7 | 7 | 7 | 7 | 7 | 7 |
| Hexyl Laurate | 4 | 4 | 4 | 4 | 4 | 4 | 4 | 4 | 4 | 4 |
| PVP/Eicosene Copolymer | 2 | 2 | 2 | 2 | 2 | 2 | 2 | 2 | 2 | 2 |
| Propylene Glycol | 4 | 4 | 4 | 4 | 4 | 4 | 4 | 4 | 4 | 4 |
| Magnesium Sulfate | 0,6 | 0,6 | 0,6 | 0,6 | 0,6 | 0,6 | 0,6 | 0,6 | 0,6 | 0,6 |
| Tocopherol | 0,5 | 0,5 | 0,5 | 0,5 | 0,5 | 0,5 | 0,5 | 0,5 | 0,5 | 0,5 |
| Tocopheryl Acetate | 0,5 | 0,5 | 0,5 | 0,5 | 0,5 | 0,5 | 0,5 | 0,5 | 0,5 | 0,5 |
| Cyclomethicone | 0,5 | 0,5 | 0,5 | 0,5 | 0,5 | 0,5 | 0,5 | 0,5 | 0,5 | 0,5 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Water | ad 100 | ad 100 | ad 100 | ad 100 | ad 100 | ad 100 | ad 100 | ad 100 | ad 100 | ad 100 |

Tabelle 1 (Fortsetzung)

| | 1-11 | 1-12 | 1-13 | 1-14 | 1-15 | 1-16 | 1-17 | 1-18 |
|---|---|---|---|---|---|---|---|---|
| T-Oleo | 3 | 5 | 2 | 4 | 3 | 1 | 2 | 5 |
| Benzylidene malonate polysiloxane | | 1 | 0,5 | | | | | |
| Methylene Bis-Benztriazolyl Tetramethylbutylphenol | 1 | 1 | 0,5 | | | | | |
| Dihydroxyavcetone | 5 | 3 | 2 | 5 | 1 | 3 | 7 | 2 |
| Polyglyceryl-3-Dimerate | 3 | 3 | 3 | 3 | | | | |
| Cera Alba | 0,3 | 0,3 | 0,3 | 0,3 | 2 | 2 | 2 | 2 |
| Hydrogenated Castor Oil | 0,2 | 0,2 | 0,2 | 0,2 | | | | |
| Paraffinium Liquidum | 7 | 7 | 7 | 7 | | | | |
| Caprylic/Capric Triglyceride | 7 | 7 | 7 | 7 | | | | |
| Hexyl Laurate | 4 | 4 | 4 | 4 | | | | |
| PVP/Eicosene Copolymer | 2 | 2 | 2 | 2 | | | | |
| Propylene Glycol | 4 | 4 | 4 | 4 | | | | |
| Magnesium Sulfate | 0,6 | 0,6 | 0,6 | 0,6 | | | | |
| Tocopherol | 0,5 | 0,5 | 0,5 | 0,5 | | | | |
| Tocopheryl Acetate | 0,5 | 0,5 | 0,5 | 0,5 | 1 | 1 | 1 | 1 |
| Cyclomethicone | 0,5 | 0,5 | 0,5 | 0,5 | | | | |
| Propylparabene | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 |
| Dicocoyl Pentyerythrityl Citrate (and) Sorbitan Sesquioleate (and) Cera Alba (and) Aluminium Stearate | | | | | 6 | 6 | 6 | 6 |
| PEG-7 Hydrogenated Castor Oil | | | | | 1 | 1 | 1 | 1 |
| Zinc Stearate | | | | | 2 | 2 | 2 | 2 |
| Oleyl Erucate | | | | | 6 | 6 | 6 | 6 |
| Decyl Oleate | | | | | 6 | 6 | 6 | 6 |
| Dimethicone | | | | | 5 | 5 | 5 | 5 |
| Tromethamine | | | | | 1 | 1 | 1 | 1 |
| Glycerin | | | | | 5 | 5 | 5 | 5 |

| | | | | | | | | |
|---|---|---|---|---|---|---|---|---|
| Allantoin | | | | | 0,2 | 0,2 | 0,2 | 0,2 |
| water | ad 100 | ad 100 | ad 100 | ad 100 | ad 100 | ad 100 | ad 100 | ad 100 |

Tabelle 1 (Fortsetzung)

| | 1-19 | 1-20 | 1-21 | 1-22 | 1-23 | 1-24 | 1-25 | 1-26 | 1-27 | 1-28 | 1-29 |
|---|---|---|---|---|---|---|---|---|---|---|---|
| T-Oleo | 1 | 2 | 5 | 1 | 3 | 4 | 5 | 2 | 3 | 3 | 3 |
| Benzylidene malonate polysiloxane | | | | 1 | | | | | 1 | 1 | |
| Methylene Bis-Benztriazolyl Tetramethylbutylphenol | | | | | | 1 | 2 | 1 | | | 1 |
| Zinc oxide | | | | | | | | 5 | 2 | | |
| UV-Pearl OMC | 5 | 5 | 5 | 5 | 7 | 5 | 5 | 5 | 5 | 5 | 8 |
| UV-Pearl , OCR | | 10 | | | | | | | | | 5 |
| UV-Pearl, EthylhexylDimethylPABA | | | 10 | | | | | | | | |
| UV-Pearl, Homosalate | | | | 10 | | | | | | | |
| UV-Pearl, Ethylhexyl salicylate | | | | | 10 | | | | | | |
| UV-Pearl , OMC, BP-3 | | | | | | 10 | | | | | |
| UV-Pearl , OCR, BP-3 | | | | | | | 10 | | | | |
| UV-Pearl, Ethylhexyl Dimethyl PABA, BP-3 | | | | | | | | 10 | | | |
| UV-Pearl, Homosalate, BP-3 | | | | | | | | | 10 | | |
| UV-Pearl, Ethylhexyl salicylate, BP-3 | | | | | | | | | | 10 | |
| Butylmethoxydibenzoylmethane | | | | | | | | | | | 2 |
| UV-Pearl OMC, 4-Methyl-benzylidene Camphor | 25 | | | | | | | | | | |
| Polyglyceryl-3-Dimerate | 3 | 3 | 3 | 3 | 3 | 3 | 3 | 3 | 3 | 3 | 3 |
| Cera Alba | 0,3 | 0,3 | 0,3 | 0,3 | 0,3 | 0,3 | 0,3 | 0,3 | 0,3 | 0,3 | 0,3 |
| Hydrogenated Castor Oil | 0,2 | 0,2 | 0,2 | 0,2 | 0,2 | 0,2 | 0,2 | 0,2 | 0,2 | 0,2 | 0,2 |
| Paraffinium Liquidum | 7 | 7 | 7 | 7 | 7 | 7 | 7 | 7 | 7 | 7 | 7 |
| Caprylic/Capric Triglyceride | 7 | 7 | 7 | 7 | 7 | 7 | 7 | 7 | 7 | 7 | 7 |
| Hexyl Laurate | 4 | 4 | 4 | 4 | 4 | 4 | 4 | 4 | 4 | 4 | 4 |
| PVP/Eicosene Copolymer | 2 | 2 | 2 | 2 | 2 | 2 | 2 | 2 | 2 | 2 | 2 |
| Propylene Glycol | 4 | 4 | 4 | 4 | 4 | 4 | 4 | 4 | 4 | 4 | 4 |
| Magnesium Sulfate | 0,6 | 0,6 | 0,6 | 0,6 | 0,6 | 0,6 | 0,6 | 0,6 | 0,6 | 0,6 | 0,6 |
| Tocopherol | 0,5 | 0,5 | 0,5 | 0,5 | 0,5 | 0,5 | 0,5 | 0,5 | 0,5 | 0,5 | 0,5 |
| Tocopheryl Acetate | 0,5 | 0,5 | 0,5 | 0,5 | 0,5 | 0,5 | 0,5 | 0,5 | 0,5 | 0,5 | 0,5 |
| Cyclomethicone | 0,5 | 0,5 | 0,5 | 0,5 | 0,5 | 0,5 | 0,5 | 0,5 | 0,5 | 0,5 | 0,5 |
| Propylparabene | 0,05 | 0,05 | 0,05 | 0,05 | 0,05 | 0,05 | 0,05 | 0,05 | 0,05 | 0,05 | 0,05 |

| | | | | | | | | | | | |
|---|---|---|---|---|---|---|---|---|---|---|---|
| Methylparabene | 0,15 | 0,15 | 0,15 | 0,15 | 0,15 | 0,15 | 0,15 | 0,15 | 0,15 | 0,15 | 0,15 |
| Water | ad 100 | | | | | | | | | | |

Tabelle 2: O/W-Emulsionen, Zahlen in Gew.-%

| | 2-1 | 2-2 | 2-3 | 2-4 | 2-5 | 2-6 | 2-7 | 2-8 | 2-9 | 2-10 |
|---|---|---|---|---|---|---|---|---|---|---|
| T-Oleo | 3 | 2 | 5 | 2 | 5 | 2 | 5 | 2 | 5 | 3 |
| Methylene Bis-Benztriazolyl Tetramethylbutylphenol | | | | | | 1 | 2 | 1 | | |
| Butylmethoxydibenzoylmethane | 3 | 3 | 3 | 3 | 3 | 3 | 3 | 3 | 3 | 3 |
| 2-(4-Hydroxy-3,5-dimethoxy-benzyliden)-malonsäure-bis-(2-ethyl-hexyl)ester | 1 | 5 | 4 | | 6 | | 7 | | 2 | 1 |
| 4-Methylbenzyliden Camphor | 2 | | 3 | | 4 | | 3 | | 2 | |
| Stearyl Alcohol (and) Steareth-7 (and) Steareth-10 | 3 | 3 | 3 | 3 | 3 | 3 | 3 | 3 | 3 | 3 |
| Glyceryl Stearate (and) Ceteth-20 | 3 | 3 | 3 | 3 | 3 | 3 | 3 | 3 | 3 | 3 |
| Microwax | 1 | 1 | 1 | 1 | 1 | 1 | 1 | 1 | 1 | 1 |
| Cetearyl Octanoate | 11,5 | 11,5 | 11,5 | 11,5 | 11,5 | 11,5 | 11,5 | 11,5 | 11,5 | 11,5 |
| Caprylic/Capric Triglyceride | 6 | 6 | 6 | 6 | 6 | 6 | 6 | 6 | 6 | 6 |
| Oleyl Oleate | 6 | 6 | 6 | 6 | 6 | 6 | 6 | 6 | 6 | 6 |
| Propylene Glycol | 4 | 4 | 4 | 4 | 4 | 4 | 4 | 4 | 4 | 4 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Tromethamine | | | 1,8 | | | | | | | |
| Water | ad 100 | ad 100 | ad 100 | Ad 100 | ad 100 | ad 100 | ad 100 | ad 100 | ad 100 | ad 100 |

Tabelle 2 (Fortsetzung)

| | 2-11 | 2-12 | 2-13 | 2-14 | 2-15 | 2-16 | 2-17 | 2-18 |
|---|---|---|---|---|---|---|---|---|
| T-Oleo | 3 | 1 | 2 | 5 | 4 | 3 | 2 | 5 |
| Benzylidene malonate polysiloxane | | 1 | 0,5 | | | | | |
| Methylene Bis-Benztriazolyl Tetramethylbutylphenol | 1 | 1 | 0,5 | | | | | |
| Butylmethoxydibenzoylmethane | 3 | 3 | 3 | 3 | 3 | 3 | 3 | 3 |
| 2-(4-Hydroxy-3,5-dimethoxy-benzyliden)-malonsäure-bis-(2-ethyl-hexyl)ester | 1 | 5 | 4 | | 6 | | 7 | |
| Zinc oxide | | | 2 | | | | | |
| UV-Pearl , OMC | 15 | 15 | 15 | 30 | 30 | 30 | 15 | 15 |
| 4-Methylbenzyliden Camphor | | | | 3 | | | | |
| Phenylbenzimidazole Sulfonic Acid | | | | | 4 | | | |
| Stearyl Alcohol (and) Steareth-7 (and) Steareth-10 | 3 | 3 | 3 | 3 | | | | |
| Glyceryl Stearate | 3 | 3 | 3 | 3 | | | | |
| Microwax | 1 | 1 | 1 | 1 | | | | |
| Cetearyl Octanoate | 11,5 | 11,5 | 11,5 | 11,5 | | | | |
| Caprylic/Capric Triglyceride | 6 | 6 | 6 | 6 | 14 | 14 | 14 | 14 |
| Oleyl Oleate | 6 | 6 | 6 | 6 | | | | |
| Propylene Glycol | 4 | 4 | 4 | 4 | | | | |
| Glyceryl Stearate SE | | | | | 6 | 6 | 6 | 6 |
| Stearic Acid | | | | | 2 | 2 | 2 | 2 |
| Persea Gratissima | | | | | 8 | 8 | 8 | 8 |
| Propylparabene | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 |
| Tromethamine | | | | | 1,8 | | | |
| Glycerin | | | | | 3 | 3 | 3 | 3 |
| Water | ad 100 | ad 100 | Ad 100 | ad 100 | ad 100 | ad 100 | ad 100 | ad 100 |

Tabelle 2 (Fortsetzung)

| | 2-19 | 2-20 | 2-21 | 2-22 | 2-23 | 2-24 | 2-25 | 2-26 | 2-27 | 2-28 |
|---|---|---|---|---|---|---|---|---|---|---|
| T-Oleo | 10 | 5 | 7 | 8 | 2 | 1 | 3 | 3 | 6 | 2 |
| Benzylidene malonate polysiloxane | 1 | 2 | | | | 1 | 1 | | 1 | 0,5 |
| Butylmethoxydibenzoyl-methane | 3 | 3 | 3 | 3 | 3 | 3 | 3 | 3 | 3 | 3 |
| Octocrylene | 1 | 5 | 4 | | 6 | | 7 | | 2 | 1 |
| Methylene Bis-Benztriazolyl Tetramethylbutylphenol | | | 1 | 2 | 1 | | | 1 | 1 | 0,5 |
| Zinc oxide | | | | | 5 | 2 | | | | 2 |
| UV-Pearl , OMC | 15 | 15 | 15 | 15 | 15 | 15 | 15 | 15 | 15 | 15 |
| Caprylic/Capric Triglyceride | 14 | 14 | 14 | 14 | 14 | 14 | 14 | 14 | 14 | 14 |
| Glyceryl Stearate SE | 6 | 6 | 6 | 6 | 6 | 6 | 6 | 6 | 6 | 6 |
| Stearic Acid | 2 | 2 | 2 | 2 | 2 | 2 | 2 | 2 | 2 | 2 |
| Persea Gratissima | 8 | 8 | 8 | 8 | 8 | 8 | 8 | 8 | 8 | 8 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Glycerin | 3 | 3 | 3 | 3 | 3 | 3 | 3 | 3 | 3 | 3 |
| Water | ad 100 | ad 100 | ad 100 | Ad 100 | ad 100 | ad 100 | ad 100 | ad 100 | ad 100 | ad 100 |

## Patentansprüche

1. Ölige Dispersion eines nanopartikulären UV-Schutzmittels, welches eine Siliciumdloxid-Beschichtung aufweist, **dadurch gekennzeichnet, dass** die Dispersion 30 bis 70 Gew.-% eines Esters von gesättigten unverzweigten Alkancarbonsäuren einer Kettenlänge von 6 bis 12 C-Atomen mit Butylenglykol und
   2 bis 15 Gew.-% Dispergierhilfsmittel enthaltend Polyglyceryldipolyhydroxystearat und
   25 bis 60 Gew.-% nanopartikuläres UV-Schutzmittel mit Siliciumdioxid-Beschichtung enthält.

2. Ölige Dispersion eines nanopartikulären UV-Schutzmittels nach Anspruch 1, **dadurch gekennzeichnet, dass** das Dispergierhilfsmittel vorzugsweise in Mengen von 2,5 bis 10 Gew.-%, insbesondere bevorzugt in Mengen von 3 bis 6 Gew.-% bezogen auf die gesamte Dispersion in der Dispersion enthalten ist.

3. Ölige Dispersion eines nanopartikulären UV-Schutzmittels nach mindestens einem der vorstehenden Ansprüche, **dadurch gekennzeichnet, dass** es sich bei dem Ester um Butylenglykoldicaprylat/dicaprat handelt, wobei der Ester vorzugsweise in Mengen von 40 bis 65 Gew.-%, insbesondere bevorzugt in Mengen von 50 bis 60 Gew.-% bezogen auf die gesamte Dispersion, in der Dispersion enthalten ist.

4. Ölige Dispersion eines nanopartikulären UV-Schutzmittels nach mindestens einem der vorstehenden Ansprüche,

**dadurch gekennzeichnet, dass** die Dispersion mindestens einen bei 25˚C festen organischen UV Filter, vorzugsweise mindestens ein Dibenzoylmethan Derivat und insbesondere bevorzugt Methoxy-tert.-butyldibenzoytmethan enthält, wobei der mindestens eine bei 25˚C feste organische UV Filter vorzugsweise in Mengen von 1 bis 20 Gew.-% bezogen auf die gesamte Dispersion, in der Dispersion enthalten ist

**5.** Ölige Dispersion eines nanopartikulären UV-Schutzmittels nach mindestens einem der vorstehenden Ansprüche, **dadurch gekennzeichnet, dass** ein nanopartikuläres UV-Schutzmittel erhältlich durch hydrothermale Behandlung eines nanopartikulären Metalloxids und anschließendes Aufbringen einer Siliciumdioxid-Beschichtung eingesetzt wird, wobei es sich bei dem Metalloxid vorzugsweise im wesentlichen um Titandioxid handelt, das gegebenenfalls mit Eisen dotiert sein kann, wobei das Titandioxid mit Siliciumdioxid-Beschichtung in der Dispersion vorzugsweise in Mengen von 30 bis 50 Gew.-%, insbesondere bevorzugt in Mengen von 33 bis 40 Gew.- % bezogen auf die gesamte Dispersion, in der Dispersion enthalten ist

**6.** Ölige Dispersion eines nanopartikulären UV-Schutzmittels nach mindestens einem der vorstehenden Ansprüche, **dadurch gekennzeichnet, dass** die Kristallitgröße des nanopartikulären Metalloxides in dem nanopartikulären UV-Schutzmittel bestimmt nach der Scherrer-Methode im Bereich von 5 nm bis 100 nm, vorzuzugsweise im Bereich 8 bis 50 nm und insbesondere bevorzugt unterhalb von 25 nm liegt und die im Transmissionselektronenmikroskop ermittelbaren Abmessungen des nanopartikulären Metalloxides bei einer Länge von 5 bis 150 nm und einer Breite von 5 bis 60 nm, vorzugsweise bei einer Länge im Bereich von 20 bis 60 nm und einer Breite im Bereich von 8 bis 30 nm liegen und das nanopartikuläre UV-Schutzmittel eine Partikelgröße nach der Scherrer-Methode im Bereich von 5 nm bis 100 nm, vorzuzugsweise im Bereich 8 bis 50 nm und insbesondere bevorzugt unterhalb von 25 nm aufweist und die im Transmissionselektronenmikroskop ermittelbaren Abmessungen des nanopartikulären UV-Schutzmittels bei einer Länge von 5 bis 160 nm und einer Breite von 10 bis 70 nm, vorzugsweise bei einer Länge im Bereich von 30 bis 70 nm und einer Breite im Bereich von 18 bis 40 nm liegen.

**7.** Ölige Dispersion eines nanopartikulären UV-Schutzmittels nach mindestens einem der vorstehenden Ansprüche, **dadurch gekennzeichnet, dass** die Siliciumdioxid-Beschichtung bezogen auf das nanopartikuläre UV-Schutzmittel 5 bis 50 Gew.-%, vorzugsweise 8 bis 30 Gew.-% und insbesondere bevorzugt 12 bis 20 Gew.-% beträgt.

**8.** Verfahren zur Herstellung einer öligen Dispersion eines nanopartikulären UV-Schutzmittels nach mindestens einem der vorstehenden Ansprüche, **dadurch gekennzeichnet, dass** ein Ester von gesättigten und/oder ungesättigten, verzweigten und/oder unverzweigten Alkancarbonsäuren einer Kettenlänge von 6 bis 12 C-Atomen mit Butylenglykol mit dem Dispergierhilfsmittel Polyglyceryldipolyhydroxystearat und einem pulverförmigen nanopartikulären UV-Schutzmittel mit Siliciumdioxid-Beschichtung vermischt wird.

**9.** Verfahren nach Anspruch 8, **dadurch gekennzeichnet, dass** eine Vormischung aus dem Ester und dem Dispergierhilfsmittel hergestellt wird und die Vormischung mit pulverförmigen nanopartikulären UV-Schutzmittel mit Siliciumdioxid-Beschichtung vermischt wird.

**10.** Zubereitung mit Lichtschutzeigenschaften enthaltend mindestens eine ölige Dispersion eines nanopartikuläres UV-Schutzmittels nach mindestens einem der Ansprüche 1 bis 7 oder mindestens eine ölige Dispersion eines nanopartikuläres UV-Schutzmittels hergestellt nach einem Verfahren entsprechend mindestens einem der Ansprüche 8 oder 9.

**11.** Zubereitung mit Lichtschutzeigenschaften nach Anspruch 10, **dadurch gekennzeichnet, dass** es sich um eine topisch anwendbare Zubereitung, vorzugsweise eine kosmetische oder dermatologische Formulierung handelt.

**12.** Zubereitung mit Lichtschutzeigenschaften nach mindestens einem der vorstehenden Ansprüche, **dadurch gekennzeichnet, dass** die Zubereitung mindestens einen organischen UV-Filter, vorzugsweise ein Dibenzoylmethanderivat, insbesondere Methoxy-tert.-butyldibenzoylmethan, und/oder ein Benzophenon-Derivat, wie 2-Hydroxy-4-methoxybenzophenon, enthält.

**13.** Zubereitung mit Lichtschutzeigenschaften nach mindestens einem der vorstehenden Ansprüche, **dadurch gekennzeichnet, dass** die Zubereitung mindestens einen Selbstbräuner, vorzugsweise Dihydroxyacton oder ein Dihydroxyacetonderivat enthält.

**14.** Zubereitung mit Lichtschutzeigenschaften nach mindestens einem der vorhergehenden Ansprüche, **dadurch gekennzeichnet, dass** die Zubereitung mindestens einen Photostabilisator, vorzugsweise entsprechend der Formel III

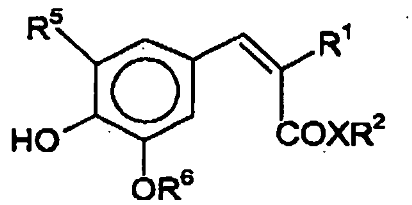

III,

wobei
$R^1$ ausgewählt ist aus $-C(O)CH_3$, $-CO_2R^3$, $-C(O)NH_2$ and $-C(O)N(R^4)_2$;
X is O or NH;
$R^2$ steht für einen linearen oder verzweigten $C_{1-30}$-Alkylrest;
$R^3$ steht für einen linearen oder verzweigten $C_{1-20}$-Alkylrest,
alle $R^4$ unabhängig voneinander stehen für H oder lineare oder verzweigte $C_{1-8}$-Alkylreste
$R^5$ steht für H, einen linearen oder verzweigten $C_{1-8}$-Alkylrest oder einen linearen oder verzweigten $-O-C_{1-6}$-Alkylrest und
$R^6$ steht für einen $C_{1-8}$-Alkylrest,
wobei es sich bei dem Photostabilisator insbesondere bevorzugt um 2-(4-Hydroxy-3,5-dimethoxy-benzyliden)-malonsäure-bis-(2-ethyl-hexyl)ester handelt, enthält.

**15.** Zubereitung mit Lichtschutzelgenschaften nach mindestens einem der vorhergehenden Ansprüche, **dadurch gekennzeichnet, dass** die Zubereitung einen oder mehrere weitere UV-Filter enthält, die vorzugsweise ausgewählt sind aus der Gruppe, die 3-(4'-Methylbenzyliden)-dl-kampfer, Methoxyzimtsäureoctylester, 3,3,5-Trimethyl-cyclohexylsalicylat, 4-(Dimethylamino)benzoesäure2-ethylhexylester, 2-Cyano-3,3-diphenylacrylsäure-2-ethylhexylester, 2-Phenylbenzimidazol-5-sulfonsäure sowie ihre Kalium-, Natrium- und Triethanolaminsalze enthält.

**16.** Zubereitung mit Lichtschutzeigenschaften nach mindestens einem der vorhergehenden Ansprüche geeignet zum Schutz von Körperzellen gegen oxidativen Stress, insbesondere zur Verringerung der Hautalterung,
**dadurch gekennzeichnet, dass** sie vorzugsweise ein oder mehrere Antioxidantien enthält.

**17.** Zubereitung mit Lichtschutzeigenschaften nach mindestens einem der vorstehenden Ansprüche, **dadurch gekennzeichnet, dass** es sich um eine Emulsion, vorzugsweise eine Öl-in-Wasser-Emulsion handelt.

**18.** Verfahren zur Herstellung einer Zubereitung nach mindestens einem der vorstehenden Ansprüche 10 bis 17 **dadurch gekennzeichnet, dass** mindestens eine ölige Dispersion eines nanopartikuläres UV-Schutzmittels nach mindestens einem der Ansprüche 1 bis 7 oder mindestens eine ölige Dispersion eines nanopartikuläres UV-Schutzmittels hergestellt nach einem Verfahren entsprechend mindestens einem der Ansprüche 8 oder 9 mit einem kosmetisch oder dermatologisch geeignetem Träger und ggf. weitern Inhaltstoffen vermischt wird.

**19.** Verwendung mindestens einer öligen Dispersion eines nanopartikuläres UV-Schutzmittels nach mindestens einem der Ansprüche 1 bis 7 oder mindestens einer öligen Dispersion eines nanopartikuläres UV-Schutzmittels hergestellt nach einem Verfahren entsprechend mindestens einem der Ansprüche 8 oder 9 zur Herstellung einer Zubereitung mit Lichtschutzeigenschaften.

**20.** Verwendung mindestens einer öligen Dispersion eines nanopartikuläres UV-Schutzmittels nach mindestens einem der Ansprüche 1 bis 7 oder mindestens einer öligen Dispersion eines nanopartikuläres UV-Schutzmittels hergestellt nach einem Verfahren entsprechend mindestens einem der Ansprüche 8 oder 9 zur Einarbeitung in Anstrichstoffe, Beschichungssysteme, Folien, Verpackungen, Fasern, Textilien und Formteile aus Kautschuk oder Silikonkautschuk, wie Reifen oder Isolatoren.

## Claims

**1.** Oily dispersion of a nanoparticulate UV protection agent which has a silicon dioxide coating, **characterised in that** the dispersion comprises 30 to 70% by weight of an ester of saturated unbranched alkanecarboxylic acids having a chain length of 6 to 12 C atoms with butylene glycol and
2 to 15% by weight of dispersion aid comprising polyglyceryl dipolyhydroxystearate, and

25 to 60% by weight of nanoparticulate UV protection agent having a silicon dioxide coating.

**2.** Oily dispersion of a nanoparticulate UV protection agent according to Claim 1, **characterised in that** the dispersion aid is preferably present in the dispersion in amounts of 2.5 to 10% by weight, particularly preferably in amounts of 3 to 6% by weight, based on the entire dispersion.

**3.** Oily dispersion of a nanoparticulate UV protection agent according to at least one of the preceding claims, **characterised in that** the ester is butylene glycol dicaprylate/dicaprate, where the ester is preferably present in the dispersion in amounts of 40 to 65% by weight, particularly preferably in amounts of 50 to 60% by weight, based on the entire dispersion.

**4.** Oily dispersion of a nanoparticulate UV protection agent according to at least one of the preceding claims, **characterised in that** the dispersion comprises at least one organic UV filter which is solid at 25˚C, preferably at least one dibenzoylmethane derivative and particularly preferably methoxy-tert-butyldibenzoylmethane, where the at least one organic UV filter which is solid at 25˚C is preferably present in the dispersion in amounts of 1 to 20% by weight, based on the entire dispersion.

**5.** Oily dispersion of a nanoparticulate UV protection agent according to at least one of the preceding claims, **characterised in that** use is made of a nanoparticulate UV protection agent obtainable by hydrothermal treatment of a nanoparticulate metal oxide and subsequent application of a silicon dioxide coating, where the metal oxide is preferably essentially titanium dioxide, which may optionally be doped with iron, where the titanium dioxide having a silicon dioxide coating is preferably present in the dispersion in amounts of 30 to 50% by weight, particularly preferably in amounts of 33 to 40% by weight, based on the entire dispersion.

**6.** Oily dispersion of a nanoparticulate UV protection agent according to at least one of the preceding claims, **characterised in that** the crystallite size of the nanoparticulate metal oxide in the nanoparticulate UV protection agent, determined by the Scherrer method, is in the range from 5 nm to 100 nm, preferably in the range from 8 to 50 nm and particularly preferably below 25 nm, and the dimensions of the nanoparticulate metal oxide, which can be determined in a transmission electron microscope, are at a length of 5 to 150 nm and a width of 5 to 60 nm, preferably at a length in the range from 20 to 60 nm and a width in the range from 8 to 30 nm, and the nanoparticulate UV protection agent has a particle size, according to the Scherrer method, in the range from 5 nm to 100 nm, preferably in the range from 8 to 50 nm and particularly preferably below 25 nm, and the dimensions of the nanoparticulate UV protection agent, which can be determined in a transmission electron microscope, are at a length of 5 to 160 nm and a width of 10 to 70 nm, preferably at a length in the range from 30 to 70 nm and a width in the range from 18 to 40 nm.

**7.** Oily dispersion of a nanoparticulate UV protection agent according to at least one of the preceding claims, **characterised in that** the silicon dioxide coating is 5 to 50% by weight, preferably 8 to 30% by weight and particularly preferably 12 to 20% by weight, based on the nanoparticulate UV protection agent.

**8.** Process for the preparation of an oily dispersion of a nanoparticulate UV protection agent according to at least one of the preceding claims, **characterised in that** an ester of saturated and/or unsaturated, branched and/or unbranched alkanecarboxylic acids having a chain length of 6 to 12 C atoms with butylene glycol is mixed with the dispersion aid polyglyceryl dipolyhydroxystearate and a pulverulent nanoparticulate UV protection agent having a silicon dioxide coating.

**9.** Process according to Claim 8, **characterised in that** a premix of the ester and the dispersion aid is prepared, and the premix is mixed with pulverulent nanoparticulate UV protection agent having a silicon dioxide coating.

**10.** Preparation having light-protection properties, comprising at least one oily dispersion of a nanoparticulate UV protection agent according to at least one of Claims 1 to 7 or at least one oily dispersion of a nanoparticulate UV protection agent prepared by a process corresponding to at least one of Claims 8 and 9.

**11.** Preparation having light-protection properties according to Claim 10, **characterised in that** it is a preparation which can be applied topically, preferably a cosmetic or dermatological formulation.

**12.** Preparation having light-protection properties according to at least one of the preceding claims, **characterised in that** the preparation comprises at least one organic UV filter, preferably a dibenzoylmethane derivative, in particular

methoxy-tert-butyldibenzoylmethane, and/or a benzophenone derivative, such as 2-hydroxy-4-methoxybenzophenone.

**13.** Preparation having light-protection properties according to at least one of the preceding claims, **characterised in that** the preparation comprises at least one self-tanning agent, preferably dihydroxyacetone or a dihydroxyacetone derivative.

**14.** Preparation having light-protection properties according to at least one of the preceding claims, **characterised in that** the preparation comprises at least one photostabiliser, preferably conforming to the formula III

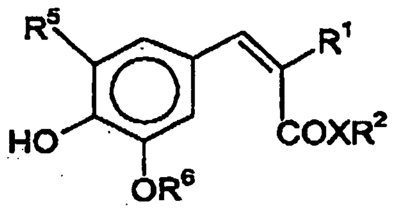

III,

where
$R^1$ is selected from $-C(O)CH_3$, $-CO_2R^3$, $-C(O)NH_2$ and $-C(O)N(R^4)_2$;
X is 0 or NH;
$R^2$ stands for a linear or branched $C_{1-30}$-alkyl radical;
$R^3$ stands for a linear or branched $C_{1-20}$-alkyl radical;
all $R^4$ stand, independently of one another, for H or linear or branched $C_{1-8}$-alkyl radicals;
$R^5$ stands for H, a linear or branched $C_{1-8}$-alkyl radical or a linear or branched $-O-C_{1-8}$-alkyl radical; and
$R^6$ stands for a $C_{1-8}$-alkyl radical;
where the photostabiliser is particularly preferably bis(2-ethylhexyl) 2-(4-hydroxy-3,5-dimethoxybenzylidene) malonate.

**15.** Preparation having light-protection properties according to at least one of the preceding claims, **characterised in that** the preparation comprises one or more further UV filters, which are preferably selected from the group comprising 3-(4'-methylbenzylidene)-dl-camphor, octyl methoxycinnamate, 3,3,5-trimethylcyclohexyl salicylate, 2-ethylhexyl 4-(dimethylamino)benzoate, 2-ethylhexyl 2-cyano-3,3-diphenylacrylate, 2-phenylbenzimidazole-5-sulfonic acid and the potassium, sodium and triethanolamine salts thereof.

**16.** Preparation having light-protection properties according to at least one of the preceding claims, suitable for the protection of body cells against oxidative stress, in particular for reducing skin ageing, **characterised in that** it preferably comprises one or more antioxidants.

**17.** Preparation having light-protection properties according to at least one of the preceding claims, **characterised in that** it is an emulsion, preferably an oil-in-water emulsion.

**18.** Process for the preparation of a preparation according to at least one of the preceding claims 10 to 17, **characterised in that** at least one oily dispersion of a nanoparticulate UV protection agent according to at least one of Claims 1 to 7 or at least one oily dispersion of a nanoparticulate UV protection agent prepared by a process corresponding to at least one of Claims 8 and 9 is mixed with a cosmetically or dermatologically suitable vehicle and optionally further ingredients.

**19.** Use of at least one oily dispersion of a nanoparticulate UV protection agent according to at least one of Claims 1 to 7 or at least one oily dispersion of a nanoparticulate UV protection agent prepared by a process corresponding to at least one of Claims 8 and 9 for the preparation of a preparation having light-protection properties.

**20.** Use of at least one oily dispersion of a nanoparticulate UV protection agent according to at least one of Claims 1 to 7 or at least one oily dispersion of a nanoparticulate UV protection agent prepared by a process corresponding to at least one of Claims 8 and 9 for incorporation into paints, coating systems, films, packaging, fibres, textiles and rubber or silicone rubber mouldings, such as tyres or insulators.

## Revendications

1. Dispersion huileuse d'un agent nanoparticulaire anti-UV ayant un revêtement en dioxyde de silicium, **caractérisée en ce que** la dispersion comprend de 30 à 70% en poids d'un ester d'acides alcanecarboxyliques non ramifiés et saturés ayant une longueur de chaîne allant de 6 à 12 atomes de C avec le butylène glycol et
de 2 à 15% en poids d'un auxiliaire de dispersion comprenant du dipolyhydroxystéarate de polyglycéryle, et
de 25 à 60% en poids d'un agent nanoparticulaire anti-UV ayant un revêtement en dioxyde de silicium.

2. Dispersion huileuse d'un agent nanoparticulaire anti-UV selon la revendication 1, **caractérisée en ce que** l'auxiliaire de dispersion est présent de préférence dans la dispersion en des quantités allant de 2,5 à 10% en poids, particulièrement préférablement en des quantités allant de 3 à 6% en poids, sur la base de la dispersion totale.

3. Dispersion huileuse d'un agent nanoparticulaire anti-UV selon au moins l'une des revendications précédentes, **caractérisée en ce que** l'ester est le dicaprylate/dicaprate de butylène glycol, où l'ester est présent de préférence dans la dispersion en des quantités allant de 40 à 65% en poids, particulièrement préférablement en des quantités allant de 50 à 60% en poids, sur la base de la dispersion totale.

4. Dispersion huileuse d'un agent nanoparticulaire anti-UV selon au moins l'une des revendications précédentes, **caractérisée en ce que** la dispersion comprend au moins un filtre UV organique qui est solide à 25°C, préférablement au moins un dérivé de dibenzoylméthane et particulièrement préférablement le méthoxy-tertio-butyldibenzoylméthane, où le au moins un filtre UV organique qui est solide à 25°C est présent de préférence dans la dispersion en des quantités allant de 1 à 20% en poids, sur la base de la dispersion totale.

5. Dispersion huileuse d'un agent nanoparticulaire anti-UV selon au moins l'une des revendications précédentes, **caractérisée en ce qu'**on utilise un agent nanoparticulaire anti-UV pouvant être obtenu par traitement hydrothermique d'un oxyde de métal nanoparticulaire et l'application consécutive d'un revêtement en dioxyde de silicium, où l'oxyde de métal est de préférence essentiellement le dioxyde de titane, pouvant être éventuellement dopé par du fer, où le dioxyde de titane ayant un revêtement en dioxyde de silicium est présent de préférence dans la dispersion en des quantités allant de 30 à 50% en poids, particulièrement préférablement en des quantités allant de 33 à 40% en poids, sur la base de la dispersion totale.

6. Dispersion huileuse d'un agent nanoparticulaire anti-UV selon au moins l'une des revendications précédentes, **caractérisée en ce que** la taille des cristallites de l'oxyde de métal nanoparticulaire dans l'agent nanoparticulaire anti-UV, déterminée par la méthode de Scherrer, se trouve dans la plage allant de 5 nm à 100 nm, préférablement dans la plage allant de 8 à 50 nm et particulièrement préférablement inférieure à 25 nm, et les dimensions de l'oxyde de métal nanoparticulaire, pouvant être déterminées à l'aide d'un microscope électronique en transmission, sont d'une longueur allant de 5 à 150 nm et d'une largeur allant de 5 à 60 nm, préférablement d'une longueur dans la plage allant de 20 à 60 nm et d'une largeur dans la plage allant de 8 à 30 nm, et l'agent nanoparticulaire anti-UV possède une granulométrie, selon la méthode de Scherrer, dans la plage allant de 5 nm à 100 nm, préférablement dans la plage allant de 8 à 50 nm et particulièrement préférablement inférieure à 25 nm, et les dimensions de l'agent nanoparticulaire anti-UV, pouvant être déterminées à l'aide d'un microscope électronique en transmission, sont d'une longueur allant de 5 à 160 nm et d'une largeur allant de 10 à 70 nm, préférablement d'une longueur dans la plage allant de 30 à 70 nm et d'une largeur dans la plage allant de 18 à 40 nm.

7. Dispersion huileuse d'un agent nanoparticulaire anti-UV selon au moins l'une des revendications précédentes, **caractérisée en ce que** le revêtement en dioxyde de silicium va de 5 à 50% en poids, préférablement de 8 à 30% en poids et particulièrement préférablement de 12 à 20% en poids, sur la base de l'agent nanoparticulaire anti-UV.

8. Procédé de préparation d'une dispersion huileuse d'un agent nanoparticulaire anti-UV selon au moins l'une des revendications précédentes, **caractérisé en ce qu'**un ester d'acides alcanecarboxyliques saturés et/ou insaturés, ramifiés et/ou non ramifiés ayant une longueur de chaîne de 6 à 12 atomes de C avec le butylène glycol est mélangé avec l'auxiliaire de dispersion, le dipolyhydroxystéarate de polyglycéryle, et un agent nanoparticulaire anti-UV pulvérulent ayant un revêtement en dioxyde de silicium.

9. Procédé selon la revendication 8, **caractérisé en ce qu'**on prépare un mélange préliminaire de l'ester et de l'auxiliaire de dispersion, et le mélange préliminaire est mélangé avec l'agent nanoparticulaire anti-UV pulvérulent ayant un revêtement en dioxyde de silicium.

**10.** Préparation ayant des propriétés photoprotectrices, comprenant au moins une dispersion huileuse d'un agent nanoparticulaire anti-UV selon au moins l'une des revendications 1 à 7 ou au moins une dispersion huileuse d'un agent nanoparticulaire anti-UV préparée par un procédé selon au moins l'une des revendications 8 et 9.

**11.** Préparation ayant des propriétés photoprotectrices selon la revendication 10, **caractérisée en ce qu'**il s'agit d'une préparation pouvant être appliquée par voie topique, préférablement une formulation cosmétique ou dermatologique.

**12.** Préparation ayant des propriétés photoprotectrices selon au moins l'une des revendications précédentes, **caractérisée en ce que** la préparation comprend au moins un filtre UV organique, préférablement un dérivé de dibenzoylméthane, en particulier le méthoxy-tertio-butyl-dibenzoylméthane, et/ou un dérivé de benzophénone, tel que la 2-hydroxy-4-méthoxybenzophénone.

**13.** Préparation ayant des propriétés photoprotectrices selon au moins l'une des revendications précédentes, **caractérisée en ce que** la préparation comprend au moins un agent autobronzant, préférablement la dihydroxyacétone ou un dérivé de dihydroxyacétone.

**14.** Préparation ayant des propriétés photoprotectrices selon au moins l'une des revendications précédentes, **caractérisée en ce que** la préparation comprend au moins un photostabilisant, répondant de préférence à la formule III

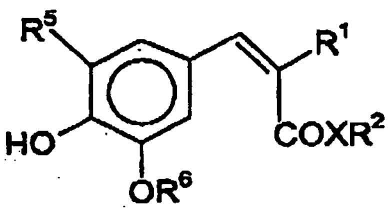

III,

dans laquelle
$R^1$ est choisi parmi $-C(O)CH_3$, $-CO_2R^3$, $-C(O)NH_2$ et $-C(O)N(R^4)_2$ ;
X est O ou NH;
$R^2$ représente un radical $C_{1-30}$-alkyle linéaire ou ramifié ;
$R^3$ représente un radical $C_{1-20}$-alkyle linéaire ou ramifié ;
tous les $R^4$ représentent, indépendamment les uns des autres, H ou des radicaux $C_{1-8}$-alkyle linéaires ou ramifiés ;
$R^5$ représente H, un radical $C_{1-8}$-alkyle linéaire ou ramifié ou un radical $-O-C_{1-8}$-alkyle linéaire ou ramifié ; et
$R^6$ représente un radical $C_{1-8}$-alkyle ;
où le photostabilisant est particulièrement préférablement le 2-(4-hydroxy-3,5-diméthoxybenzylidène)malonate de bis(2-éthylhexyl).

**15.** Préparation ayant des propriétés photoprotectrices selon au moins l'une des revendications précédentes, **caractérisée en ce que** la préparation comprend un ou plusieurs autres filtres UV, qui sont choisis de préférence parmi le groupe constitué par le 3-(4'-méthylbenzylidène)-dl-camphre, le méthoxycinnamate d'octyle, le salicylate de 3,3,5-triméthylcyclohexyle, le 4-(diméthylamino)benzoate de 2-éthylhexyle, le 2-cyano-3,3-diphénylacrylate de 2-éthylhexyle, l'acide 2-phénylbenzimidazole-5-sulfonique et les sels de potassium, sodium et triéthanolamine de ceux-ci.

**16.** Préparation ayant des propriétés photoprotectrices selon au moins l'une des revendications précédentes, convenable pour la protection des cellules de l'organisme contre un stress oxydatif, en particulier pour réduire le vieillissement de la peau, **caractérisée en ce que** qu'elle comprend de préférence one ou plusieurs antioxydants.

**17.** Préparation ayant des propriétés photoprotectrices selon au moins l'une des revendications précédentes, **caractérisée en ce qu'**il s'agit d'une émulsion, préférablement une émulsion huile-dans-eau.

**18.** Procédé de préparation d'une préparation selon au moins l'une des revendications 10 à 17 précédentes, **caractérisée en ce qu'**au moins une dispersion huileuse d'un agent nanoparticulaire anti-UV selon au moins l'une des revendications 1 à 7 ou au moins une dispersion huileuse d'un agent nanoparticulaire anti-UV préparée par un procédé selon au moins l'une des revendications 8 et 9 est mélangée avec un véhicule cosmétiquement ou dermatologiquement convenable et éventuellement d'autres ingrédients.

**19.** Utilisation d'au moins une dispersion huileuse d'un agent nanoparticulaire anti-UV selon au moins l'une des revendications 1 à 7 ou d'au moins une dispersion huileuse d'un agent nanoparticulaire anti-UV préparée par un procédé selon au moins l'une des revendications 8 et 9 de préparation d'une préparation ayant des propriétés photoprotectrices.

**20.** Utilisation d'au moins une dispersion huileuse d'un agent nanoparticulaire anti-UV selon au moins l'une des revendications 1 à 7 ou d'au moins une dispersion huileuse d'un agent nanoparticulaire anti-UV préparée par un procédé selon au moins l'une des revendications 8 et 9 pour une incorporation dans des peintures, des systèmes de revêtements, des films, des emballages, des fibres, des textiles et des pièces moulées en caoutchouc ou en caoutchouc de silicone, telles que les pneus ou les isolateurs.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- FR 2326405 A
- FR 2440933 A
- EP 0114607 A
- JP 61215314 A
- EP 0748624 A
- WO 9404131 A
- DE 10333029
- WO 9304665 A
- EP 0487404 A
- WO 9966896 A
- US 6242099 B1
- WO 0009652 A
- WO 0072806 A
- WO 0071084 A
- EP 0671161 A
- DE 4116123 A
- WO 03007906 A

### In der Beschreibung aufgeführte Nicht-Patentliteratur

- **K.Recker.** Ullmanns Enzyklopädie der Technischen Chemie. 1978, vol. 15, S.117 ff
- **E. A. Galinski et al.** *Eur. J. Biochem.,* 1985, vol. 149, 135-139
- **T. Rudolf.** UV-A-Protection assessment of Sunscreens - a critical review on the UV-A- to UV-B-absorbance ratio. *SÖFW-Journal,* 2004, vol. 130 (9), S.28ff